(19) Europäisches Patentamt
European Patent Office
Office européen des brevets

(11) **EP 1 027 863 B1**

(12) **EUROPÄISCHE PATENTSCHRIFT**

(45) Veröffentlichungstag und Bekanntmachung des Hinweises auf die Patenterteilung:
**21.01.2009 Patentblatt 2009/04**

(51) Int Cl.:
***A61B 5/12*** *(2006.01)*

(21) Anmeldenummer: **00102878.6**

(22) Anmeldetag: **11.02.2000**

(54) **Objektive Bestimmung der schwellennahen und überschwelligen Schallverarbeitung des Innenohres**

Objective determination of near and above threshold sound distorsion of the internal ear

Détermination objective de la distorsion du son auprès et au-dessus du seuil auditif

(84) Benannte Vertragsstaaten:
**AT BE CH CY DE DK ES FI FR GB GR IE IT LI LU MC NL PT SE**

(30) Priorität: **11.02.1999 DE 19905743**

(43) Veröffentlichungstag der Anmeldung:
**16.08.2000 Patentblatt 2000/33**

(73) Patentinhaber: **Path Medical GmbH**
**82110 Germering (DE)**

(72) Erfinder:
• **Thomas Janssen**
**83104 Tuntenhausen (DE)**

• **Paul Boege**
**82319 Starnberg (DE)**
• **Wolfgang Arnold**
**82131 Gauting (DE)**

(74) Vertreter: **Schwan, Ivo et al**
**Schwan Schwan Schorer**
**Patentanwälte**
**Bauerstrasse 22**
**80796 München (DE)**

(56) Entgegenhaltungen:
**US-A- 5 664 577**

## Beschreibung

Die vorliegende Erfindung betrifft ein Verfahren und eine Vorrichtung zur objektiven Bestimmung der schwellennahen und überschwelligen Schallverarbeitung des Innenohres.

Die Funktion des Gehörs kann auf zweierlei Arten gestört werden. Insbesondere können die schallaufnehmenden und schallverarbeitenden Hörsysteme, wie Mittel- und Innenohr, in ihrer Funktion derart eingeschränkt sein, dass Schallsignale zu leise (Hypoakusis), verzerrt (Hyperakusis) oder überhaupt nicht aufgenommen (Surditas) und an das Zentralnervensystem weitergeleitet werden, oder aber, das Gehör selbst kann Störsignale (Tinnitus) produzieren, die wie echte Schallsignale verarbeitet und bewusst wahrgenommen werden. Beide Symptome, Schwerhörigkeit und Tinnitus, können gemeinsam oder unabhängig voneinander auftreten. Nach Schätzungen der Weltgesundheitsorganisation leiden 350 Millionen Menschen unter Hörstörungen - das entspricht 7 % der Weltbevölkerung. Nach neuesten epidemiologischen Studien leiden etwa 10 % der Bevölkerung Europas und der Vereinigten Staaten an Tinnitus.

Etwa 90 % aller Hörstörungen sind Störungen der Innenohrfunktion. Der Diagnostik der Innenohrschwerhörigkeit kommt daher eine ganz besondere Bedeutung zu. Trotz der großen Fortschritte der Hörphysiologie in den letzten Jahrzehnten werden die der Innenohrschwerhörigkeit zugrunde liegenden Pathomechanismen noch nicht in allen Punkten verstanden. Somit sind nicht immer die Voraussetzungen für eine gezielte Behandlung (z.B. beim Tinnitus) oder für eine gezielte Prävention (z.B. Vorsorgeuntersuchungen bei Lärmarbeitern) gegeben.

Das stumme Stadium frühkindlicher Hörstörungen verläuft naturgemäß symptomarm, denn Verhaltens- und Vokalisationsmuster des hörgestörten Kindes sind bis zum siebten Lebensmonat weitgehend identisch mit denen normalhörender Säuglinge. Daher wird der Verdacht auf das Vorliegen einer Hörstörung in der Regel zu spät erhoben. Die Diskrepanz zwischen dem optimalen Therapiezeitpunkt, nämlich mindestens ab dem sechsten Lebensmonat, und der Diagnosestellung, die in Deutschland und in anderen europäischen Staaten bei zwei Jahren liegt, macht deutlich, dass alle Anstrengungen unternommen werden müssen, um eine möglichst frühe Diagnosestellung zu erreichen.

Aber auch bei Jugendlichen und Erwachsenen gewinnt mit der zunehmenden Belastung durch Lärm am Arbeitsplatz und in der Freizeit die Früherkennung von Hörstörungen immer mehr an Bedeutung. Die Gehörgefährdung durch überlaute Musik bewirkt in zunehmendem Maße Gehörschäden bei Jugendlichen, wie sie früher nur bei Lärmarbeitern auftraten. Lärm in Form zu lauter Musik, aber auch die allgemeine Lärmbelastung des Alltags führen besonders dann zu Gehörschäden, wenn eine Disposition in Form einer erhöhten Vulnerabilität des Innenohres vorliegt. Diese erhöhte Empfindlichkeit gegen Lärmbelastung kann durch perinatale Asphyxie oder andere pränatale oder frühkindliche Gesundheitsstörungen verursacht werden. Unter extremen Lärmbelastungen ist bei solchen vorgeschädigten Ohren mit besonders großen, oft bleibenden Hörverlusten und mit persistierendem Tinnitus zu rechnen.

Die akute oder chronische Überlastung des Gehörs durch Lärm bewirkt eine Funktionsstörung der äußeren Haarzellen des Cortischen Organs. Vieles deutet darauf hin, dass auch der Tinnitus als häufiges Begleitsymptom traumatischer Gehörschäden auf der Ebene der äußeren Haarzellen generiert wird. Auch beim Hörsturz, dessen Pathogenese heute noch unklar ist, wird eine Dysfunktion äußerer Haarzellen vermutet.

Da in den überwiegenden Fällen die Innenohrfunktionsstörung nicht durch medikamentöse Behandlung oder auf operativen Weg geheilt oder ausreichend gebessert werden kann, kommt ein großer Personenkreis für die Versorgung mit Hörgeräten in Betracht. Die Anzahl der in Deutschland von der Krankenkasse erstatteten Hörgeräte liegt bei über fünfhunderttausend im Jahr. Der Diagnostik und der Therapie der Innenohrschwerhörigkeit mit Hilfe von Hörgeräten kommt daher eine besondere Bedeutung zu.

Für die Hörgeräteanpassung ist die detaillierte Erfassung der Funktionsstörung des Innenohres im gesamten Frequenz- und Dynamikbereich des Hörens von entscheidender Bedeutung. Zur Einstellung der Verstärkung eines Hörgerätes muss bekannt sein, bei welcher Frequenz welcher Hörverlust auftritt (Tonschwellenverlauf) und ob ein pathologischer Lautheitsanstieg (Recruitment) vorliegt, d.h. es müssen aus den audiometrischen Testverfahren Informationen über die schwellennahe und überschwellige Schallverarbeitung gewonnen werden.

Im Innenohr erfolgt die Umsetzung des mechanischen Reizes in Nervenimpulse mit Hilfe der inneren Haarzellen. Da diese aber nur eine eingeschränkte Dynamik haben (bis 50 dB) muss zur Übertragung des gesamten wahrgenommenen Dynamikbereichs (130 dB beim Menschen) eine Dynamikkompression von 3:1 stattfinden. Dies geschieht durch die äußeren Haarzellen, deren Aufgabe es ist, kleine Eingangssignale zu verstärken, um so den inneren Haarzellen zu ermöglichen, den gesamten Dynamikbereich des Schalles zu übertragen. Wegen ihrer höheren Empfindlichkeit werden die äußeren Haarzellen stärker geschädigt als die inneren Haarzellen. Als Folge der Schädigung äußerer Haarzellen kommt es zu einem Recruitment. Recruitment ist eine veränderte Form des Lautheitsanstiegs, bei der ein großer Hörverlust bei leisen und nur ein kleiner Hörverlust bei lauten Schallen auftritt. Mit dem Recruitment ist unmittelbar auch ein Trennschärfeverlust verbunden, der zu erheblichen Einschränkungen des Sprachverstehens führt.

Durch Hintereinanderschaltung von Hörhilfe und gestörtem Gehör sollte idealerweise ein normales Hörvermögen erreicht werden. Diesem hohen Anspruch werden jedoch auch moderne digitale Hörgeräte nicht gerecht. Dies liegt aber nicht in erster Linie an den technischen Unzulänglichkeiten, sondern eher daran, dass wichtige Kenndaten der Innenohrfunktionsstörungen mit den vorhandenen diagnostischen Methoden nicht oder nur mit unzureichender Genauigkeit erfasst werden können. Üblicherweise wird die frequenz- und eingangspegelabhängige Verstärkung für Hörgeräte auf der Basis statistischer Daten, aus der Hörschwelle und gegebenenfalls aus der Unbehaglichkeitsschwelle ohne Berücksichtigung der überschwelligen Schallverarbeitung (Lautheitsanstieg) abgeschätzt.

Moderne Hörgeräte mit digitaler Signalverarbeitungstechnologie bieten die Möglichkeit zur mehrkanaligen Einstellung von Verstärkung und Kompression in bis zu 14 Frequenzbändern, wobei die Bandbreiten mit denen des realen Innenohres übereinstimmen. Um die technischen Möglichkeiten dieser Hörgeräte voll ausschöpfen zu können, müssen neue diagnostische Methoden eingesetzt werden, die in der Lage sind, in differenzierter Weise das Recruitment und den Trennschärfeverlust quantitativ und mit hoher Frequenzauflösung zu erfassen. In neuester Zeit wird zwar zur Erfassung des Lautheitsanstiegs die kategoriale Lautheitsskalierung vorgenommen, sie ist jedoch eine subjektive Methode und weist zudem nur eine grobe Frequenzauflösung auf.

Die Früherkennung einer kindlichen Schwerhörigkeit, die Früherkennung von beginnenden Lärmschädigungen, die Erkennung einer erhöhten Innenohr-Vulnerabilität, die topologische Tinnitusdiagnostik und die speziell auf die Anpassung von Hörgeräten ausgerichtete Innenohr-Diagnostik sind zentrale Themen der Audiologie. Die Audiologie stellt heute zwar audiometrische Untersuchungsmethoden bereit, die es erlauben, zwischen Mittelohr- und Innenohrschwerhörigkeit und zentralen Hörstörungen zu differenzieren, sie ist aber noch nicht in der Lage, die integrierenden Schallverarbeitungsmechanismen des Hörsystems, speziell des Innenohres, und dessen Störungen im Detail zu erfassen. Dies gilt vor allem für Säuglinge und Kleinkinder, wo nur objektive audiometrische Methoden angewendet werden können. Aber auch beim Erwachsenen reichen die aus den subjektiven Tests ermittelten Kenngrößen nicht aus, um die Innenohrschwerhörigkeit, vor allem den veränderten Lautheitsanstieg, genauer zu beschreiben.

Eine neue objektive audiometrische Untersuchungsmethode, welche die otoakustischen Emissionen nutzt, bietet zur Zeit die beste Möglichkeit zur gezielten Diagnostik des Innenohres. Otoakustische Emissionen sind Schallaussendungen des Innenohres, die mit einem empfindlichen Mikrophon im äußeren Gehörgang gemessen werden können. Sie entstehen durch die Verstärkungsaktivität der äußeren Haarzellen.

Die Existenz otoakustischer Emissionen als Epiphänomen der Verstärkung und der Trennschärfe des Innenohres wurde von Gold im Jahre 1948 postuliert. Die messtechnische Bestätigung gelang 1978 durch Kemp (siehe EP-B-0 015 258 und GB-A-2 205 403). Bisher haben sich die mit kurzen Reizimpulsen ausgelösten transitorisch evozierten otoakustischen Emissionen (TEOAE) in der klinischen Diagnostik als Methode des Hörscreenings in der Kinderhördiagnostk oder in der Differentialdiagnostik cochleärer und retrocochleärer Funktionsstörungen etablieren können.

Neben der Auslösung mit kurzen Reizimpulsen können otoakustische Emissionen auch bei stationärer Anregung mit zwei gleichzeitig dargebotenen Sinustönen (Primärtöne) der Frequenz $f_1$ und $f_2$ ($f_i<f_2$) ausgelöst werden. Diese otoakustischen Emissionen werden als Distorsionsprodukte otoakustischer Emissionen (DPOAE) bezeichnet.

Aus US-A-5 664 577 (Lonsbury-Martin et al.) ist ein Verfahren zur Erfassung sowohl schwellennaher als auch überschwelliger Schallverarbeitung des Innenohres durch Messung von mindestens Distorsionsprodukten otoakustischer Emissionen (DPOAE) bekannt, bei welchem:

(a) eine Ohrsonde in den Gehörgang des zu untersuchenden Ohres eingeführt wird;

(b) ein erster Primärton mit einer Frequenz $f_1$ und einem Schallpegel $L_1$, sowie ein zweiter mit einer Frequenz $f_2$, die größer als die Frequenz $f_1$ des ersten Primärtones ist, und einem Schallpegel $L_2$ von der Ohrsonde im äußeren Gehörgang erzeugt werden, um DPOAE auszulösen;

(c) Distorsionsprodukte otoakustischer Emissionen (DPOAE) im äußeren Gehörgang gemessen werden;

(d) die Frequenz $f_{DP}$ und der Schalldruck $p_{DP}$ bzw. der Emissionspegel $L_{DP}$ der gemessenen DPOAE ermittelt werden; und

(e) der Schalldruck $p_{DP}$ bzw. der Emissionspegel $L_{DP}$ gegenüber dem Schalldruck $L_1$ bzw. $L_2$ von einem der Töne aufgetragen wird, um eine DPOAE- Wachstumsfunktion zu erhalten.

DPOAE entstehen als direkte Folge der nichtlinearen Schallverarbeitung im Cortischen Organ, vornehmlich im Bereich der äußeren Haarzellen. DPOAE sind virtuelle, nicht im Reiz enthaltene Töne, die die Frequenzen $nf_1\pm mf_2$ haben (n und m sind ganze Zahlen). Beim Menschen hat die Emission mit der Frequenz $2f_1-f_2$ (kubischer Differenzton) die größte Amplitude.

Die Applikation der Primärtöne und die Registrierung der Emissionen erfolgt mit einer im äußeren Gehörgang platzierten Sonde. Die Sonde enthält ein Mikrophon zur Registrierung der Emission sowie Schallsender zur Erzeugung der Primärtöne.

DPOAE entstehen im Überlappungsbereich der durch die Primärtöne ausgelösten Wanderwellen auf der Basilarmembran. Im wesentlichen tragen die Haarzellen zur Schallaussendung bei, die nach der Frequenz-Orts-Transformation in der Cochlea nahe bei $f_2$ liegen. Die im Gehörgang gemessene Schallemission ist charakterisiert durch ihre Frequenz $f_{DP}$ (vorzugsweise $2f_1$-$f_2$) und ihren Schalldruck $p_{DP}$ bzw. Schalldruckpegel $L_{DP}$ ($L_{DP}$= 20 · log ($p_{DP}/p_0$) [SPL]; $p_0 = 2 \cdot 10^{-5}$ Pa). Wegen der Entstehung der Emission nahe $f_2$ wird üblicherweise der Schallpegel $L_{Dp}$ *als* Funktion von $f_2$ aufgetragen (DP-Gramm).

DPOAE lassen sich nahezu im gesamten Frequenzbereich des Hörens messen. Bei Frequenzen unterhalb $f_2$ = 500 Hz wird die Registrierung wegen der im unteren Frequenzbereich zunehmenden Störgeräusche schwieriger. Zum Erlangen maximaler Emissionspegel wird ein bestimmtes Frequenzverhältnis $f_2/f_1$ eingestellt $f_2/f_1$ variiert in Abhängigkeit von $f_2$ (etwa zwischen 1,3 und 1,1 beim Menschen). Mit abnehmendem Primärtonpegel nimmt der Emissionspegel in der Regel ab. Der Emissionspegel hängt auch von der Differenz $L_1$-$L_2$ der Primärtöne ab. Als Folge der unterschiedlichen Schallreizung (Sinuston / Transient) lassen sich im Vergleich zu den TEOAE mit den DPOAE Funktionsstörungen des Innenohres mit sehr viel höherer Frequenzauflösung erfassen.

Die uneingeschränkte Funktion der äußeren Haarzellen ist entscheidend für die hohe Sensitivität und Trennschärfe des Gehörs. Gehen die äußeren Haarzellen durch perinatale Asphyxie, Knall- oder Lärmtrauma, Hörsturz, Ototoxen oder bei der Altersschwerhörigkeit zugrunde, so sind diese für das Sprachverstehen wichtigsten Funktionen eingeschränkt. Mit Hilfe der DPOAE, lassen sich in der Art eines akustischen Cochleogramms Funktion und Dysfunktion des cochleären Verstärkungsprozesses höchst sensitiv und quantitativ erfassen. Hierdurch eröffnen sich neue Wege der allgemeinen und im speziellen auf die Anpassung mehrkanaliger digitaler Hörgeräte ausgerichteten Innenohrdiagnostik mit genauer Bestimmung des Recruitments und des Trennschärfeverlustes.

Bisher werden in der audiologischen Diagnostik die Kenngrößen zur Charakterisierung der gestörten Innenohrfunktion mit nicht aufeinander abgestimmten Methoden und mit verschiedenen Apparaturen erhoben. Im besonderen werden unterschiedliche elektroakustische Wandler (Lautsprecher, Kopfhörer und Ohrsonde) verwendet, die einen direkten Vergleich der Messgrößen nicht zulassen. Weitere Nachteile, die sich aus dieser Vorgehensweise ergeben, sind lange Untersuchungszeiten, sowie, wegen der ungenügenden Vernetzung und Kompatibilität der Messdaten, langwierige und komplizierte Diagnose-Stellungen.

Es ist daher eine Aufgabe der vorliegenden Erfindung, ein Verfahren und eine Vorrichtung zu schaffen, mittels welchen sich sowohl die schwellennahe als auch die überschwellige Schallverarbeitung des Innenohres möglichst präzise und dennoch auf einfache Weise bewerten lassen.

Nach der vorliegenden Erfindung wird diese Aufgabe durch ein Verfahren zur Erfassung sowohl schwellennaher als auch überschwelliger Schallverarbeitung des Innenohres durch Messung von mindestens Distorsionsprodukten otoakustischer Emissionen (DPOAE) gelöst, wie es in Anspruch 1 angegeben ist.

Die Erfindung ermöglicht durch Messung von Distorsionsprodukten otoakustischer Emissionen (DPOAE) eine objektive und quantitative Erfassung sowohl der schwellennahen als auch der überschwelligen Schallverarbeitung des Innenohres. Das erfindungsgemäße Verfahren und die nachfolgend beschriebene entsprechende erfindungsgemäße Vorrichtung eignen sich insbesondere: 1. zur Bestimmung der Hörschwelle, des Lautheitsanstieges und der Trennschärfe, wobei die hierbei gewonnenen Daten insbesondere zur Anpassung von Hörgeräten verwendet werden können, 2. zur Früherkennung von beginnenden Hörstörungen durch Lärmbelastung, 3. zur Erfassung einer erhöhten Innenohr-Vulnerabiliät, 4. zur Diagnostik des cochleären Tinnitus und 5. zur therapiebegleitenden Verlaufskontrolle beim Hörsturz, Lärmtrauma und bei Verabreichung ototoxischer Medikamente. Nachdem das erfindungsgemäße Verfahren, bei dem es sich in erster Linie um ein Auswerteverfahren von durch Applikation von Schallreizen angeregten Schallaussendungen des Innenohres handelt, keinerlei Gesundheitsrisiken für den Probanden mit sich bringt, ist kein medizinisches Fachpersonal zur Durchführung oder Beaufsichtigung des vorgeschlagenen Verfahrens erforderlich. Da eine erhöhte Lärmanfälligkeit des Probanden erfasst und durch Lärmbelastung verursachte Hörstörungen bereits im Anfangsstadium erkannt werden können, kann eine ernstzunehmende Schädigung des Hörapparats oftmals durch präventive Maßnahmen, beispielsweise durch Vorgabe bestimmter Verhaltensweisen, insbesondere Lärmvermeidung, oder durch Umgestaltung des Arbeitsplatzes des Probanden, bereits im Vorfeld vermieden werden. Darüber hinaus eignet sich das erfindungsgemäße Verfahren auch zur Anwendung bei Reihenuntersuchungen.

Weitere Merkmale und Vorteile des vorgeschlagenen Verfahrens sowie der in den Unteransprüchen angegebenen bevorzugten Ausführungsformen desselben sind weiter unten detailliert erläutert;

Mit der vorliegenden Erfindung wird ferner eine zur Ausführung des vorgeschlagenen Verfahrens geeignete Vorrichtung zur Erfassung sowohl schwellennaher als auch überschwelliger Schallverarbeitung des Innenohres und seiner Störungen durch Messung von mindestens Distorsionsprodukten otoakustischer Emissionen (DPOAE) bereitgestellt, wie sie in Anspruch 14 angegeben ist.

Durch Messung der DPOAE im äußeren Gehörgang und drahtlose Übertragung der Messdaten zu einer Auswerteanordnung werden Störgeräusche und die damit verbundenen Messfehler, wie sie bei bisher bekannten Messanordnungen, bei welchen die Ohrsonde in einer direkten Kabelverbindung mit der Auswerteanordnung steht, wirkungsvoll vermieden. Bei den bekannten Messanordnungen wurden die Messungen schon durch kleinste Bewegungen entweder des Probanden oder von Teilen des Messvorrichtung selbst hervorgerufen, da zur Erfassung der DPOAE hochempfindliche Mikrophone eingesetzt werden, die neben den Messsignalen jegliche Störgeräusche registrieren, wie sie z.B.

auftreten, wenn ein zu den Ohrsonden führendes Kabel eine Bewegung erfährt, beispielsweise wenn es aufgrund einer Kopfbewegung des Probanden gegen eine Oberfläche, wie beispielsweise ein Kleidungsstück des Probanden, reibt. Die Erfindung ermöglicht somit erstmals eine weitgehend messfehlerfreie objektive und quantitative Erfassung sowohl der schwellennahen als auch der überschwelligen Schallverarbeitung des Innenohres.

Bevorzugte Ausgestaltungen der vorgeschlagenen Vorrichtung ergeben sich aus den Unteransprüchen.

Insbesondere kann mindestens eine Elektrodenanordnung zur zusätzlichen Erfassung von frühen auditorisch evozierten Potentialen (FAEP) vorgesehen sein, um eine Bestimmung der Hörschwelle auch bei hochgradigen Hörverlusten zu ermöglichen, so dass eine Erfassung der Innenohrfunktionsstörung im gesamten Dynamikbereich ermöglicht wird. In diesem Fall kann ferner die tragbare Einheit eine Verbindungsanordnung zum lösbaren Verbinden der Elektrodenanordnung mit der tragbaren Einheit aufweisen. Die Verbindungsanordnung kann z.B. an der tragbaren Einheit vorgesehene Buchsen sowie Stecker für die Elektroden umfassen.

Des weiteren kann die tragbare Einheit eine Verstärkeranordnung zur Verstärkung der von dem Mikrophon erfassten DPOAE-Signale sowie gegebenenfalls der mittels der Elektrodenanordnung erfassten FAEP-Signale umfassen. Vorzugsweise kann auch eine Digitalisierungseinrichtung vorgesehen sein, um die erfassten Signale, insbesondere nachdem sie verstärkt wurden, zu digitalisieren, bevor sie mittels dem drahtlosen Sende- und Empfangssystem, bei dem es sich beispielsweise um ein Infrarotsystem oder ein Funksystem handeln kann, zu der stationären Einheit übertragen werden. Dies trägt erheblich zur Fehlersicherheit bei der Datenübermittlung bei.

Gemäß einer bevorzugten Ausführungsform der Erfindung ist die tragbare Einheit als ein am Kopf eines Probanden zu tragender Halter, insbesondere als Kopf- oder Kinnbügel (Headset), ausgelegt. Durch die vorgenannten Maßnahmen lässt sich die erfindungsgemäße Vorrichtung zur Erfassung der Schallverarbeitung des Innenohres als eine kleine, leicht zu handhabende Einheit ausbilden, die alle zur Auslösung und Messung der DPOAE und falls erwünscht der FAEP benötigten Einheiten umfasst, wobei die Ansteuerung sowie die eigentliche Auswertung der erfassten Daten an der von der tragbaren Einheit räumlich getrennten, jedoch mit dieser in Datenverbindung stehenden stationären Einheit, wie beispielsweise einem Rechner (DSP bzw. PC), Notebook oder Palmtop, erfolgt.

Die zur Auslösung der DPOAE und FAEP benutzten Schallsender können einen ersten Schallsender zur Abgabe eines ersten Primärtones, einen zweiten Schallsender zur Abgabe eines zweiten Primärtones, sowie falls erwünscht einen dritten Schallsender zur Abgabe eines Suppressortones umfassen, die alle in der Ohrsonde untergebracht sind. Die Messung der DPOAE kann dabei mit einem Mikrophon, oder falls erwünscht zwecks Reduzierung des Mikrophonrauschens und damit zwecks Verbesserung des Signal-Störabstandes mit mehreren parallelgeschalteten Mikrophonen erfolgen, die alle in der Ohrsonde untergebracht sind.

Vorzugsweise wird ein spezielles Reizparadigma eingesetzt, welches der nichtlinearen Schallverarbeitung der beiden Primärtöne am primären Ort der Entstehung der DPOAE, bei $f_2$, Rechnung trägt und welches damit die schwellennahe Messung der DPOAE und somit die Erfassung der Innenohrfunktion und seiner Störungen in einem erweiterten Dynamikbereich erlaubt.

In weiterer Ausgestaltung der Erfindung kann die Stromversorgung eine nachladbare Stromquelle aufweisen, wobei dann vorzugsweise ferner eine Ladestation zum Nachladen der nachladbaren Stromquelle vorgesehen ist, beispielsweise eine Halterung, in welche die tragbare Einheit eingelegt oder eingehängt werden kann, wobei an der tragbaren Einheit und an der Ladestation Kontakte vorgesehen sind, die, sobald die tragbare Einheit und die Ladestation miteinander in Eingriff treten, für eine elektrische Verbindung zwischen diesen sorgen.

Zusätzlich zu der tragbaren Einheit kann ferner ein tragbarer Signalgeber vorgesehen sein, der eine Eingabevorrichtung sowie ein Sendesystem zur drahtlosen Übertragung von mittels der Eingabevorrichtung erzeugten Signalen zu der stationären Einheit aufweist. Sollen mit der erfindungsgemäßen Vorrichtung zusätzlich zu den vorliegend beschriebenen objektiven Gehöruntersuchungen subjektive Hörtests durchgeführt werden, so kann ein derartiger Signalgeber als Eingabevorrichtung einen Tastschalter aufweisen, mit welchem ein Proband Signale an die stationäre Einheit übermitteln kann, beispielsweise um der stationären Einheit anzuzeigen, wann bei einer Untersuchung, bei welchem das zu untersuchende Ohr ausgehend von einem unterschwelligen Reiz mit steigendem Reizpegel beschallt wird, die subjektive Hörschwelle erreicht ist.

Die stationäre Einheit kann eine erste Speicher- und Wiedergabeanordnung zur Speicherung und Wiedergabe von während eines Messvorgangs ermittelten Daten aufweisen, sowie eine zweite Speicher- und Wiedergabeanordnung zur Speicherung und Wiedergabe eines Programms zur Steuerung eines Messvorgangs. Letzteres kann außer Maschinenbefehlen, wie z.B. Steuerbefehlen zur Ansteuerung der Schallsender, auch Anweisungen an einen Probanden umfassen, so dass sich weitgehend automatisierte Messabläufe realisieren lassen.

Bevorzugte Ausführungsbeispiele der Erfindung werden im folgenden unter Bezugnahme auf die beiliegenden Zeichnungen im Detail beschrieben. Es zeigen:

FIG. 1    eine schematische Ansicht einer Ausführungsform der erfindungsgemäßen Vorrichtung, mit der sich die erfindungsgemäßen Verfahren durchführen lassen,

FIG. 2    eine Ausführungsform der Darstellung von DP-Grammen, dem DP-Steigungsprofil und der DPOAE-Wachstumsfunktion zur Erläuterung der erfindungsgemäßen Vorgehensweise zur Erfassung der Sensitivität und Dynamikkompression des Innenohres und seiner Störungen am Beispiel eines Patients mit Hochtonhörverlust,

FIG. 3 die erfindungsgemäße Vorgehensweise zur Ermittlung der Kenndaten aus den DPOAE-Iso-Suppressionstuningkurven zur Erfassung der Trennschärfe des Innenohres und seiner Störungen,

FIG. 4 die erfindungsgemäße Vorgehensweise zur Bestimmung des Primärtonschwellenpegels mittels extrapolierten DPOAE-Wachstumsfunktionen,

FIG. 5 die erfindungsgemäße Vorgehensweise zur Darstellung der aus dem Primärtonschwellenpegel $L_S$ rekonstruierten Hörschwelle und der aus den Iso-Emissionspegel-Kurven gewonnenen Dynamikkompression im konventionellen Tonschwellenaudiogramm am Beispiel eines Patients mit Hochtonhörverlust, und

FIG. 6 eine schematische Schnittansicht durch ein Ausführungsbeispiel einer Ohrsonde, wie sie bei der in FIG. 1 gezeigten Vorrichtung eingesetzt wird.

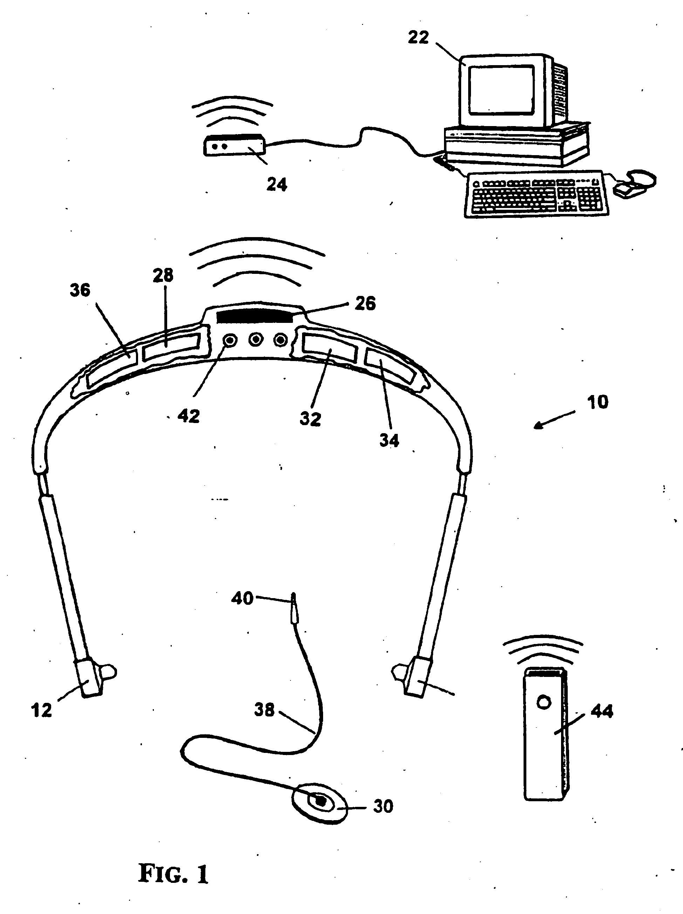

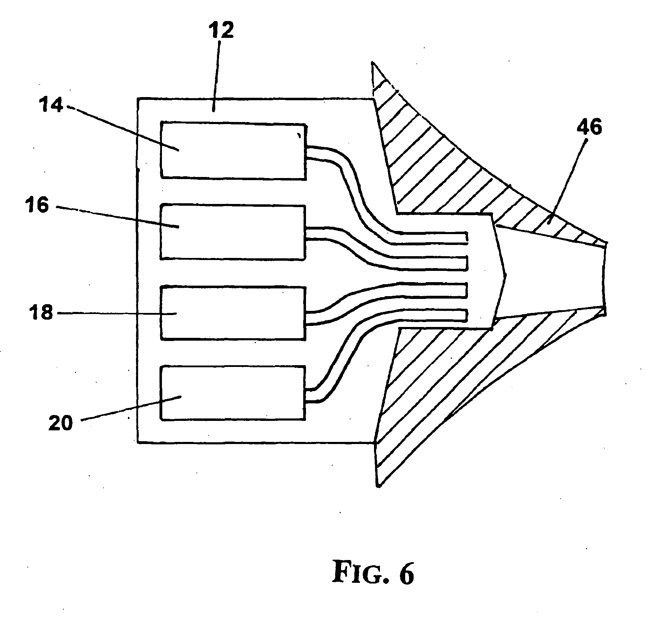

Die vorliegende beschriebene Vorrichtung zur Erfassung sowohl der schwellennahen als auch der überschwelligen Schallverarbeitung des Innenohres weist entsprechend FIG. 1 einen Kopf- bzw. Kinnbügel (Headset) 10 auf, der mindestens eine, vorzugsweise jedoch zwei Ohrsonden 12 trägt, die jeweils mit einer Ohrolive 46 (FIG. 6) versehen sind, die in den Gehörgang des zu untersuchenden Ohrs derart einpassbar ist, dass es diesen luftdicht abschließt. Wie aus der schematischen Ansicht gemäß FIG. 6 zu entnehmen ist, sind in jeder der Ohrsonden 12 ein erster Schallsender 14 zur Abgabe eines ersten Primärtones und ein zweiter Schallsender 16 zur Abgabe eines zweiten Primärtones zur Auslösung der DPOAE sowie ein Mikrophon 20 zur Registrierung der DPOAE angeordnet. Soll ferner ein Suppressorton appliziert werden, so kann in den Ohrsonden 12 ein zusätzlicher dritter Schallsender 18 vorgesehen sein. Die Applikation des Suppressortones kann jedoch auch mittels einer der primärtonaussendenden Schallsender erfolgen. Es können in den Ohrsonden 12 auch mehrere Mikrophone vorgesehen sein, um das Gesamt-Mikrophonrauschen zu reduzieren.

Mit dem ersten Schallsender 14 und/oder dem zweiten Schallsender 16 lassen sich zudem auch FAEP sowie, durch Applikation transienter Schallreize, TEOAE auslösen. Der in FIG. 1 dargestellte Kopfhalter 10 ist mit Buchsen 42 ausgestattet, in die auf Zuleitungen 38 von Kopfhautelektroden 30 angebrachte Stecker 40 eingesteckt werden können, um die Kopfhautelektroden 30 mit der in dem Kopfhalter 10 untergebrachten Elektronik, die im folgenden näher erläutert wird, zu verbinden.

Zur besseren Handhabung der Vorrichtung bei Säuglingen und Kleinkindern kann eine Trennung von Ohrsonden 12 und Kopfbügel 10 vorgenommen werden. Die Ohrsonden 12 und der Kopfbügel 10 werden dann mit Kabeln miteinander verbunden.

Die Steuerung des Messablaufs sowie die Analyse und Dokumentation der Daten erfolgt über einen Rechner 22 (bzw. ein DSP, Notebook oder Palmtop), der Signale zum Ansteuern der Schallsender, wie in FIG. 1 gezeigt, über ein drahtloses Sende- und Empfangssystem 24, insbesondere ein Infrarot-Datenübertragungssystem, an den Kopf- bzw. Kinnbügel 10 übermittelt. Die mittels einer entsprechenden Sende- und Empfangseinheit 26 des Kopf- bzw. Kinnbügels 10 empfangenen Signale werden dann mittels ebenfalls in dem Kopf- bzw. Kinnbügel 10 untergebrachten Verstärkern 28 zur Erzeugung der Schallreize (Sinustöne bei DPOAE und FAEP, Impulse bei FAEP und TEOAE) umgesetzt und an die in den Ohrsonden 12 untergebrachten Schallsender 14, 16 und 18 weitergeleitet. Die Verstärker 28 ebenso wie die weiteren nachstehend beschriebenen, in dem Kopf- bzw. Kinnbügels 10 untergebrachten elektrischen und elektronischen Komponenten sind zur Vereinfachung der Darstellung in FIG. 1 nur schematisch als Blöcke innerhalb der Abschnitte des Kopf- bzw. Kinnbügels 10 dargestellt, die durch die teilweise weggebrochene Ansicht freigelegt sind, um einen Blick in das Innere des Kopf- bzw. Kinnbügels 10 zu gewähren.

Durch die von den Schallsendern 14, 16 und 18 applizierten Schallreize ausgelöste DPOAE und FAEP werden mittels dem in der jeweiligen Ohrsonde 12 angeordneten Mikrophon 20 sowie mit Elektroden 30, die auf der Kopfhaut des Probanden angeordnet werden, registriert. Die registrierten Signale (Mikrophonspannung bei DPOAE, Elektrodenspannung bei FAEP) werden durch einen in dem Kopf- bzw. Kinnbügel 10 untergebrachten Verstärker 32 verstärkt, mittels einer Digitalisieranordnung 34 digitalisiert und mittels der Sende- und Empfangseinheit 26 an den Rechner 22 zur Auswertung und Archivierung der Daten übermittelt.

An der Hörschwelle (bei 2 kHz) beträgt der Emissionsschalldruckpegel $L_{DP}$ etwa -40 dB SPL L=20•log (p/p$_0$), p$_0$=2•10$^{-5}$ Pa). Dies entspricht etwa einem Hundertstel des die Emission auslösenden Reizpegels. Wegen der sehr kleinen Amplituden wird die Emission zumindest bei kleinen Reizpegeln vom Störgeräusch (Mikrophonrauschen, Atemgeräusch, Blutrauschen) überlagert. Zur Messung dieser sehr kleinen Schalldrücke und zur Verbesserung des Signalstörabstandes werden daher ein rauscharmes Mikrophon 20, ein geeigneter Mikrophonverstärker 32 und ein Mittelungsverfahren im Zeitbereich eingesetzt.

Um die in dem Kopf- bzw. Kinnbügel 10 untergebrachten elektronischen Einheiten mit Strom zu versorgen, ist in dem Kopf- bzw. Kinnbügel 10 ferner eine Stromversorgung 36, vorzugsweise eine nachladbare Stromversorgung, untergebracht. Aufgrund der Abkoppelung der die Schallreize auslösenden Komponenten und der die DPOAE, sowie gegebenenfalls FAEP und/oder TEOAE, registrierenden Komponenten von der Netzspannung ist die Patientensicherheit im Vergleich zu bekannten Vorrichtungen erhöht. Durch die drahtlose Übertragung der Messdaten, die zuvor digitalisiert wurden, kann die Störanfälligkeit bei der Datenübertragung reduziert werden.

Soll mit der vorliegend beschriebenen Vorrichtung auch die Durchführung der subjektiven Methoden der konventionellen Audiometrie wie Tonschwellenaudiogramm, Sprachaudiogramm, überschwellige Tests (z.B. Fowler und SISI) und eine Lautheitsskalierung durch Implementierung möglich sein, so werden eine Patiententaste 44 zur Aufnahme der subjektiven Tonschwelle sowie ein Eingabenfeld für die Lautheitsskalierung mit integrierten Systemen zur Datenübertragung vorgesehen.

Die Messabläufe sowie die Datenanalyse erfolgen basierend auf einem in dem Rechner 22 ablaufenden Programm weitgehend automatisch und vorzugsweise unter Einsatz von Algorithmen und Kriterien zur Artefakterkennung und Qualitätssicherung. Neben der getrennten Messung von DPOAE und FAEP können zur Beschleunigung der Messdatenaufnahme und zur Verkürzung der Untersuchungszeit die DPOAE und FAEP simultan, d.h. bei gleichzeitiger Reizapplikation, gemessen werden.

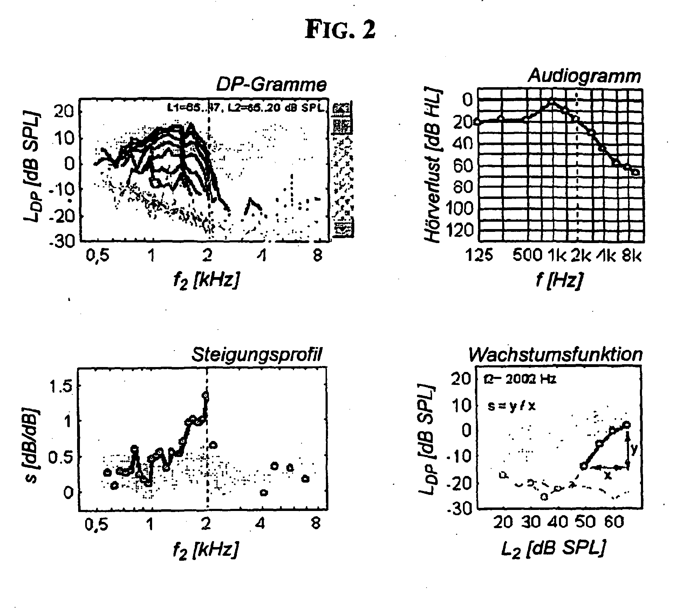

Die vorliegend beschriebene Messapparatur erlaubt die Messung der DPOAE, sowie bei der bevorzugten Ausgestaltung der hier beschriebenen Vorrichtung der FAEP, und die Bereitstellung der daraus abgeleiteten Kenngrößen der Innenohrfunktion und seiner Störungen gemäß den nachstehend unter Bezugnahme auf die FIG. 2 bis 5 beschriebenen Methoden.

*1. Erfassung der Kompression*

Bisher gelingt es nur mit invasiven elektrophysiologischen oder optischen Methoden (im Tiermodell) direkte Aussagen über die Verstärkung und Trennschärfe des Innenohres zu treffen. Die DPOAE bieten erstmalig die Möglichkeit, auch beim Menschen die Verstärkung und Trennschärfe des Innenohres nicht-invasiv und quantitativ zu erfassen. Dies gelingt am besten dann, wenn die die DPOAE auslösenden Primärtöne in Frequenz und Pegel so variiert werden, dass die entstehenden nichtlinearen Verzerrungen tatsächlich die Merkmale der Verstärkung und Trennschärfe des Innenohres widerspiegeln. Hierzu kommt ein Reizparadigma zur Anwendung, welches der unterschiedlichen Dynamikkompression der beiden Primärtöne am Ort ihrer Entstehung, bei $f_2$, Rechnung trägt. Die Primärtöne werden gemäß der Formel (1)

$$L_1 = \sum_{n=0}^{N} a_n L_2{}^n \qquad \qquad \text{(I)}$$

bei einem festen Frequenzverhältnis $f_2/f_1=c$ ($f_2>f_1$) im Bereich zwischen $L_2$ = -10 bis +70 dB SPL eingestellt. Eine bevorzugte Parametereinstellung ist hierbei $N = 1$, $25 \leq a_0 \leq 50$ und $0{,}3 \leq a_1 \leq 0{,}6$. Die Applikation von Primärtönen mit größeren Schalldruckpegeln wäre nicht nur denkbar sondern auch durchaus wünschenswert, ist jedoch mit derzeit verfügbaren Schallsendern und Mikrophonen nicht realisierbar. Eine mögliche Einstellung der Parameter zur Erzeugung maximaler Emissionspegel ist: $a_0$ = 39, $a_1$ = 0,4, $c$ = 1,2. Die Parameter $a_0$, $a_1$ und $c$ können in Abhängigkeit vom Cochleaort, respektive $f_2$, oder individuell variieren.

Zur Erfassung der kompressiven Verstärkungseigenschaften des Innenohres und seiner Störungen wird der gemessene Emissionspegel $L_{DP}$ bzw. der Schalldruck $p_{DP}$ als Funktion des Schallpegels eines der Primärtöne (vorzugsweise $L_2$) aufgetragen. Hierdurch entsteht die DPOAE-Wachstumsfunktion $L_{DP}(L_2)$ bzw. $p_{DP}(L_2)$. Als Maß für die Kompression dient das Kompressionsverhältnis k = $\Delta L_2 / \Delta L_{DP}$. Dies ist die Änderung des Primärtonpegels in dB (vorzugsweise $L_2$), die notwendig ist, um eine Änderung des Emissionspegels $L_{DP}$ von einem dB zu erreichen. Ein weiteres Maß ist die vorzugsweise im Primärtonpegelbereich zwischen $L_2$ = 40 und $L_2$ = 60 dB SPL berechnete Steigung $s$ der DPOAE-Wachstumsfunktion (Reziprokwert des Kompressionsverhältnisses). Zur Erfassung der ortsabhängigen Dynamikkompression wird das Kompressionsverhältnis bzw. die Steigung der an verschiedenen Cochleaorten aufgenommenen DPOAE-Wachstumsfunktionen als Funktion der Primärtonfrequenz (vorzugsweise $f_2$) aufgetragen. Die so erhaltene Funktion ist das DPOAE-Steigungsprofil $s(f_2)$ bzw. Kompressionsprofil $k(f_2)$.

Bei normaler Hörfunktion spiegeln die am Menschen mit dem Reizparadigma $L_1 = \sum_{n=0}^{N} a_n L_2{}^n$, insbesondere mit $L_1 = a_0 + a_1 L_2$, aufgenommenen DPOAE-Wachstumsfunktionen die aus Tiermodellen bekannte kompressive nichtlineare Schallverarbeitung wieder, mit Steigungen von im Mittel 0,8 dB/dB im unteren Reizpegelbereich ($L_2$ < 25dB) und 0,1 dB/dB im oberen Reizpegelbereich ($L_2$ > 60dB). Mit zunehmendem Hörverlust kommt es zu einer Linearisierung des Wachstums des Emissionspegels mit Steigungen von bis über 1 dB/dB im oberen Reizpegelbereich (Janssen et al. in "Growth behavior of the 2f1-f2 distortion product otoacoustic emission in tinnitus", J. Acoust. Soc. Am. 103 (6), Seiten 3418 bis 3430, Juni 1998; Kummer et al. in "The level and growth behavior of the 2f1-f2 distortion product otoacoustic emission and its relationship to auditory sensitivity in normal hearing and cochlear hearing loss", J. Acoust. Soc. Am. 103 (6), Seiten 3431 bis 3444, Juni 1998). Das bedeutet, der Hörverlust drückt sich in der Abnahme des Emissionspegels, der pathologische Lautheitsanstieg (Recruitment) in der Versteilerung der Wachstumsfunktion aus.

Das vorliegend beschriebene Verfahren verwendet als Kenngröße zur Erfassung des Hörverlustes die Abweichung des Emissionspegels $L_{DP}$ vom Normwert, als Kenngröße zur Erfassung des pathologischen Lautheitsanstiegs (Recruitment) die Abweichung der Steigung $s(f_2)$ bzw. des Kompressionsverhältnisses $k(f_2)$ von der Norm (vgl. FIG. 2).

Somit werden neue Kenngrößen bereitgestellt, mit deren Hilfe die Erfassung des Lautheitsanstiegs an beliebigen Orten der Cochlea (Frequenzen) möglich ist. Damit wird eine individuelle Einstellung des sprachrelevanten Einsatzpunkts der Kompression und die Einstellung der Kompression in den verschiedenen Frequenzkanälen eines Hörgerätes ermöglicht. Bisher steht kein objektives Verfahren zur an den individuellen Verstärkungsbedarf angepassten Einstellung von Hörgerätes Verfügung. Es sei darauf hingewiesen, dass sich das vorstehend beschriebene Verfahren ebenso wie die nachfolgend beschriebenen Mess- und Auswerteverfahren, wenngleich aufgrund der dann auftretenden Störsignale erheblich schlechter, auch mit einem System durchführen lassen, bei welchem die Datenübertragung zwischen der Ohrsonde und der Auswerteanordnung über Kabel erfolgt. Wie zuvor erwähnt, führen aufgrund der äußerst niedrigen Emissionspegel der DPOAE jegliche Bewegungen des Probanden, die notwendigerweise dann auch zu einer Reibung an den die Ohrsonden und die Auswerteeinheit verbindenden Kabeln führt, zu einer Störung der Messsignale. Eine Datenübertragung zwischen der tragbaren Einheit und der stationären Einheit mittels Kabeln wäre angesichts der Ergebnisse, die sich mit der vorliegend beschriebenen drahtlosen Datenübertragung erreichen lassen, allenfalls dann tolerierbar, wenn derartige Bewegungen ausgeschlossen oder auf ein Mindestmaß begrenzt sind, beispielsweise im Tierversuch, wenn das zu untersuchende Tier ruhig gestellt bzw. betäubt ist.

Die bei den verschiedenen Primärtonpegeln aufgenommenen DP-Gramme werden, wie in FIG. 2 oben links gezeigt, in einem Plot dargestellt, wobei, z.B. über eine Scroll-Taste am Bildschirm, die DP-Gramme auch einzeln gezeigt werden können. Für jedes DP-Gramm wird der Normbereich (z.B. als 2-fache Standardabweichung) eingetragen. Das gemessene Störgeräusch des Patienten (bzw. Probanden) gibt zusammen mit dem Mittelwert (ggf. auch der Standardabweichung) des Störgeräuschs eines Normalkollektivs Auskunft über das individuell erzeugte Störgeräusch. Mit Hilfe eines Cursors (gestrichelte Linie) kann die Frequenz selektiert werden, für die die DPOAE-Wachstumsfunktion angezeigt werden soll. Im Plot der Wachstumsfunktion (FIG. 2 unten rechts) kann so für jede gewählte Frequenz der Verlauf der Wachstumsfunktion gezeigt werden. Das aus dem Verlauf der Wachstumsfunktion berechnete Kompressionsverhältnis $k$ bzw. die Steigung $s = 1/k$ wird für jede Wachstumsfunktion angegeben, wobei der Pegelbereich wählbar ist. In einem separaten Plot wird das Kompressionsverhältnis $k$ bzw. die Steigung $s = 1/k$ als Funktion der Frequenz aufgetragen (FIG. 2, unten links). Auch hier wird der Normbereich der Messgrößen angezeigt (grau-schattierter Bereich in FIG. 2). Das entsprechende Tonschwellenaudiogramm ist in FIG. 2 oben rechts dargestellt.

*2. Erfassung der Trennschärfe*

Bietet man dem Ohr mittels Ohrsonde zusätzlich zu den Primärtönen einen dritten Ton, den Suppressorton, mit der Frequenz $f_{sup}$ und dem Schallpegel $L_{sup}$ an, und ermittelt bei verschiedenen Frequenzen $f_{sup}$ im Bereich der DPOAE-Frequenz $f_{DP}$ den Schallpegel $L_{sup}$, der zu einer Suppression des Emissionspegels $L_{DP}$ um einen bestimmten Wert (z.B. 4 dB) führt, so erhält man eine DPOAE-Iso-Suppressionstuningkurve (Kummer et al. in "Suppression tuning characteristics of the 2f1-f2 distortion product otoacoustic emission in humans", J. Acoust. Soc. Am. 98 (1), Seiten 197 bis 210, Juli 1995).

DPOAE-Iso-Suppressionstuningkurven weisen die gleichen Merkmale auf, wie die aus den Tiermodellen bekannten neuralen Tuningkurven. Damit ist die Möglichkeit einer objektiven und nicht-invasiven Erfassung der Trennschärfeeigenschaften des Innenohres gegeben. Bisher werden in der klinischen Diagnostik Trennschärfedefizite und die damit verbundenen Diskriminationsverluste nur mit subjektiven Methoden, in der Regel mit Hilfe des Sprachaudiogramms, bestimmt. In der Erwachsenen-Hördiagnostik bieten sich die DPOAE daher als Ergänzung zum Sprachaudiogramm an. In der Kinderhördiagnostik sind sie die einzige Methode zur Bestimmung der Trennschärfe des Gehörs.

Das vorliegend beschriebene Verfahren verwendet zur Erfassung der Innenohr-Trennschärfe und seiner Störungen die Kenngrößen der DPOAE-Iso-Suppressionstuningkurve. Das sind vorzugsweise die Steigungen der tieffrequenten und der hochfrequenten Tuningkurvenflanke $s_{tf}$ und $s_{hf}$, die charakteristische Frequenz $f_{cf}$ der Tuningkurve, der Emissionspegel $L_{DPCF}$ bei $f_{DF}$, und die Trennschärfe $Q_{10dB}$. Die Einstellung der Primärtonpegel erfolgt auch hier vorzugsweise nach der Formel (I)

$$L_I = \sum_{n=0}^{N} a_n L_2^{\,n} \qquad\qquad\qquad (I),$$

insbesondere mit $N$=1 und $f_2/f_1 = c$.

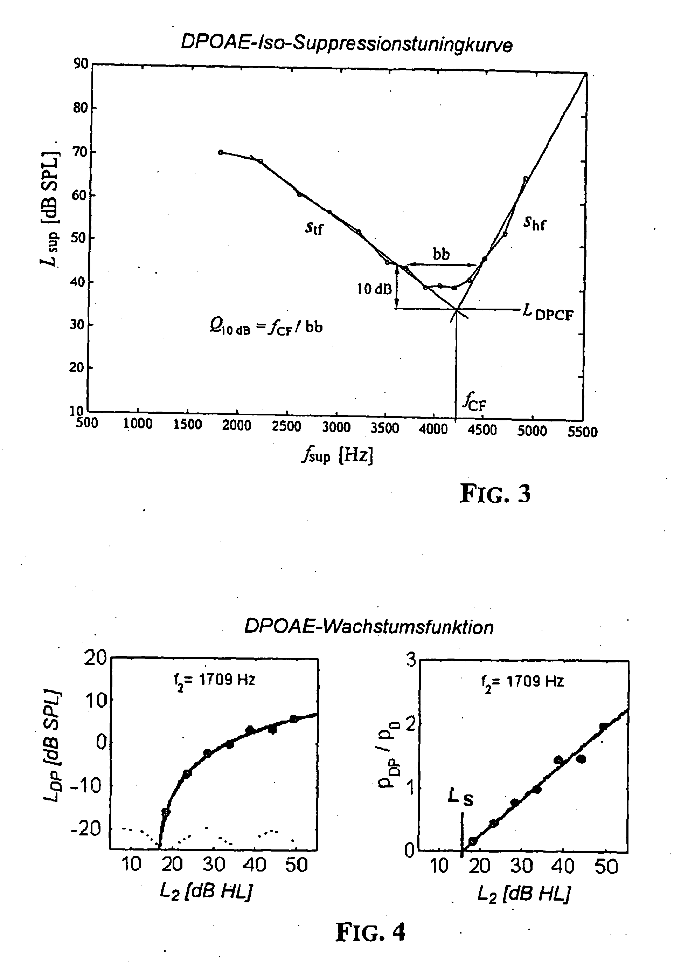

FIG. 3 zeigt ein Beispiel zur Ermittlung der Kenndaten aus den DPOAE-Iso-Suppressionstuningkurven zur Erfassung der Trennschärfe des Innenohres und seiner Störungen. Die tief- und die hochfrequente Flanke der Tuningkurve werden durch Geraden angenähert und die Steigungen $s_{tf}$ und $s_{hf}$, der durch den Schnittpunkt der Geraden bestimmte Tuningkurvenschwellenpegel $L_{DPCF}$ und die charakteristische Frequenz $f_{CF}$ sowie der $Q_{10dB}$-Wert werden ermittelt. Solche Tuningkurven werden in verschiedenen Frequenzbereichen aufgenommen.

Als weitere Methode zur Erfassung der Innenohr-Trennschärfe dient die $f_2/f_1$-Tuningkurve. Zur Erstellung der $f_2/f_1$-Tuningkurve wird der Emissionspegel $L_{DP}$, bzw. der Schalldruck $p_{DP}$ als Funktion des Frequenzverhältnisses $f_2/f_1$ aufgetragen. Das Frequenzverhältnis $f_2/f_1$ wird vorzugsweise zwischen 1,05 und 1,6 in kleinen Frequenzschrittweiten (von z.B. 1 - 100 Hz) variiert. Der Vorteil gegenüber der DPOAE-Iso-Suppressionstuningkurve liegt darin, dass zur Aufnahme der $f_2/f_1$-Tuningkurve kein zusätzlicher dritter Ton appliziert werden muss. Die spiegelbildlich aufgetragene $f_2/f_1$-Tuningkurve weist die gleichen Merkmale auf wie die DPOAE-Iso-Suppressionstuningkurve, so dass die oben beschriebenen Kenngrößen auch hier zur Erfassung der Innenohrtrennschärfe verwendet werden können.

Die hier erläuterte Vorrichtung stellt somit Kenngrößen zur Erfassung der Innenohrtrennschärfe an beliebigen Orten (Frequenzen) der Cochlea bereit. Die Kenngroßen ermöglichen eine individuelle Einstellung der Bandbreite der Frequenzkanäle von Hörgeräten

*3. Bestimmung der Hörschwelle.*

Wegen des engen Zusammenhanges zwischen Emissionspegel und Hörschwelle ist eine objektive Bestimmung der Hörschwelle mit Hilfe der DPOAE grundsätzlich möglich. Wegen der Störgeräusche ist die Messung der DPOAE jedoch bei Primärtonpegeln an der Hörschwelle überhaupt nicht oder nur mit langen Mittelungszeiten möglich. Eine weitere Problematik der Bestimmung der Hörschwelle ist das Auftreten von erhöhten Emissionspegeln bei Patienten mit Tinnitus (Janssen et al. in "Growth behavior of the 2f1-f2 distortion product otoacoustic emission in tinnitus", J. Acoust. Soc. Am. 103 (6), Seiten 3418 bis 3430, Juni 1998). Bei diesen Patienten ist eine Vorhersage der Hörschwelle alleine auf der Basis des Emissionspegels nicht möglich. Hier kann eine Bestimmung der Hörschwelle nur über das Wachstumsverhalten der DPOAE erfolgen.

Da die Emissionen an der Hörschwelle Normalhörender oder bei größeren Hörverlusten sehr kleine Amplituden aufweisen und im Mikrophonrauschen verschwinden, kann der Reizpegel, der zur Auslösung der DPOAE an der Hörschwelle führt, nicht zuverlässig ermittelt und somit aus dem die Emission erzeugenden Primärtonpegel keine Hörschwelle rekonstruiert werden.

Durch Extrapolation der mit $L_I = \sum_{n=0}^{N} a_n L_2^{\,n}$ aufgenommenen DPOAE-Wachstumsfunktionen lässt sich jedoch derjenige Primärtonpegel (vorzugsweise $L_2$) bestimmen, der zum Schalldruck $p_{DP}$=0 führt. Dieser als $L_s$ bezeich-nete Primärtonschwellenpegel dient zur direkten Bestimmung der Hörschwelle.

Wie in FIG. 4 dargestellt, wird zur Extrapolation der DPOAE-Wachstumsfunktion die rechts unten in FIG. 2 im doppellogarithmischen Plot dargestellte Wachstumsfunktion vorzugsweise im halblogarithmischen Plot dargestellt, wobei der gemessene Emissionsschalldruck $p_{DP}$ / $p_0$ über $L_2$ aufgetragen ist. Die Annäherung der Messdaten erfolgt hier durch lineare Regressionsrechnung. Der Schnittpunkt der Regressionsgeraden mit der Abszisse ist der Primärton-schwellenpegel $L_s$. Es sind aber auch andere Methoden des Messdaten-Fittings möglich.

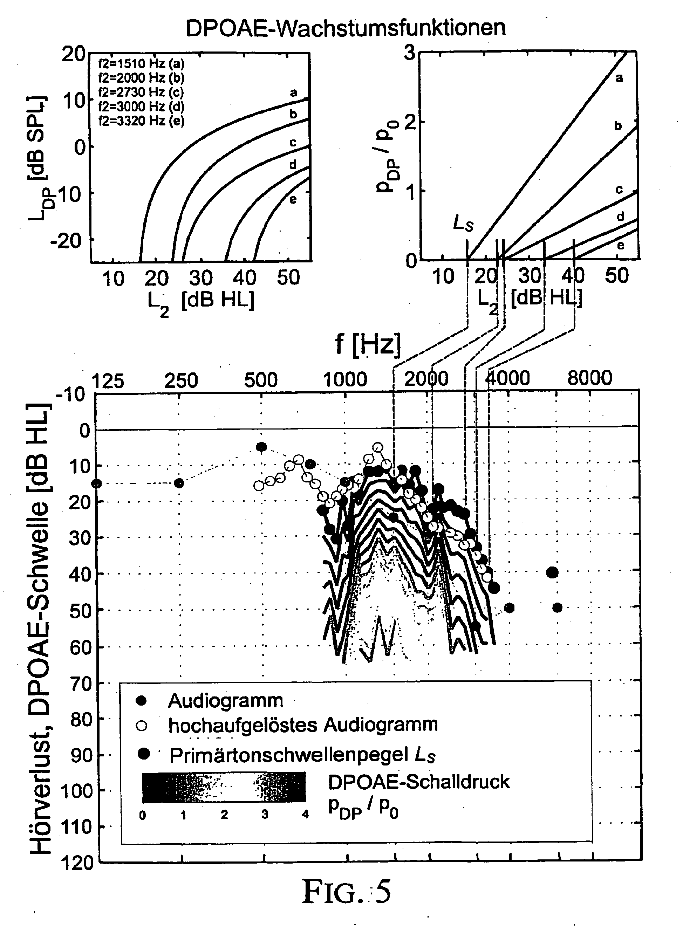

FIG. 5 zeigt die erfindungsgemäße Vorgehensweise zur Bestimmung der aus den Primärtonschwellenpegeln $L_S$ rekonstruierten Hörschwelle und der aus den Iso-Emissionspegel-Kurven gewonnenen Dynamikkompression am Beispiel eines Patienten mit Hochtonhörverlust.

In FIG. 5 oben links sind auszugsweise fünf Wachstumsfünktionen (a-e) gezeigt, die bei $f_2$ = 1510 Hz (Kurve a),$f_2$ = 2000 Hz (Kurve b),$f_2$ = 2730 Hz (Kurve c),$f_2$ = 3000 Hz (Kurve d) und $f_2$ = 3320 Hz (Kurve e) aufgenommen wurden. In FIG. 5 oben rechts sind die Regressionsgeraden der Wachstumsfunktionen dargestellt, die entsprechend mit a-e gekennzeichnet sind. Die Schnittpunkte der Regressionsgeraden mit der Abszisse liefern den Primärtonschwellenpegel $L_S$ für die jeweilige Frequenz. Berechnet man die Primärtonschwellenpegel aller Wachstumsfünktionen und trägt sie in ein klinisches Tonschwellenaudiogramm (FIG. 5 unten) ein, so erhält man eine Schwelle (geschlossene Kreise), die mit der mit gleicher Ohrsonde bei gleicher Frequenz aufgenommen subjektiven Hörschwelle (offene Kreise) gut übereinstimmt. Diese Schwelle dient der objektiven Bestimmung der Hörschwelle bei der Innenohrschwerhörigkeit.

Zusätzlich sind im Audiogramm die Kurven gleichen Schalldrucks eingetragen. Aus ihren Abständen lassen sich wie auf einer Wanderkarte mit Höhenlinien der Anstieg des DPOAE-Schalldruckes und damit die Dynamikkompression des Innenohrs für die einzelnen Frequenzen ablesen.

Der Eintrag von $L_s$ als Hörschwelle und $L_{DP}$ als Iso-Pegelkurven im konventionellen Tonschwellenaudiogramm ergibt ein DPOAE-Audiogramm und ermöglicht so die gemeinsame Darstellung der schwellennahen (Hörschwelle) und überschwelligen (Dynamikkompression) Schallverarbeitungsmechanismen des Innenohres in einem Plot.

Die Erfindung stellt eine Methode und eine Vorrichtung bereit, mit der es durch Extrapolation der DPOAE-Wachstumsfunktionen möglich ist, den Primärtonpegel zu berechnen, der zur Auslösung der DPOAE an der Hörschwelle führt. Mit dem so berechneten Schwellenpegel kann mit heutigen DPOAE-Messsystemen eine Rekonstruktion der Hörschwelle bei Hörverlusten von bis zu 60 dB HL mit geringer Fehlerbreite vorgenommen werden. Da hierbei die Berechnung der Hörschwelle ausschließlich auf Messdaten basiert, handelt es sich hierbei um eine echte objektive Methode zur Bestimmung der Hörschwelle. In der Kinderhördiagnostik fehlt bislang eine solche Methode zur Hörschwellenbestimmung.

Die otoakustischen Emissionen als Epiphänomen der Schallverarbeitung im unteren und mittleren Schallintensitätsbereich des Gehörs lassen sich jedoch bei hochgradigen Hörverlusten nicht messen. Um Information über die Schallverarbeitung des Innenohres auch bei hochgradigen Hörverlusten, d.h. größer als 60 dB, zu gewinnen, werden zur Bestimmung der Hörschwelle daher zusätzlich die bereits in der klinischen Diagnostik etablierten auditorisch evozierten Potentiale gemessen.

Die vorliegend beschriebene Vorrichtung gestattet die Registrierung der FAEP sowohl in herkömmlicher Weise bei Applikation von transienten Schallreizen (Klicks) als auch in neuer Weise, nämlich simultan bei Applikation der zur Auslösung der DPOAE verwendeten Primärtöne. Zur Erhöhung der Frequenzspezifität der FAEP kann bei beiden Reizmodalitäten Bandpassrauschen mit veränderlicher Eckfrequenz zum Ausschluss nicht interessierender Cochleabschnitte (vorzugsweise zur Eliminierung der basalen Miterregung bei hohen Reizpegeln) appliziert werden. Mit Hilfe des Primärtonschwellenpegels $L_s$ und des Latenz- und Amplitudenverhaltens der FAEP kann die Bestimmung der Hörschwelle im gesamten Hörbereich vorgenommen werden.

Die primäre Quelle der DPOAE der Frequenz $2f_1\text{-}f_2$ liegt im Überlappungsbereich der durch die beiden Primärtöne ausgelösten Wanderwellen auf der Basilarmembran am Ort der Frequenz $f_2$ des höheren Primärtones. Es existiert jedoch noch eine sekundäre Quelle mit sehr viel kleinerer Amplitude am Ort der Frequenz $2f_1\text{-}f_2$ die sich störend auf die primäre Quelle auswirkt (Heitmann et al., DE-OS 195 49 165) und somit zu einem größeren Schätzfehler bei der Vorhersage der Hörschwelle führen könnte. Der Störungsmechanismus wurde bislang nur für normalhörende Probanden beschrieben. Falls die störende Quelle auch bei der Innenohrschwerhörigkeit auftritt, kann bei der hier erläuterten Vorrichtung ein Suppressorton mit einer Frequenz nahe bei $2f_1\text{-}f_2$ zur Unterdrückung der störenden Quelle appliziert werden.

*4. Früherkennung von Innenohrfunktionsstörungen*

Wegen ihrer hohen Sensitivität und Frequenzspezifität können DPOAE kleinste pathologische Veränderungen des Innenohres erfassen; sie sind damit allen konventionellen tonaudiometrischen Verfahren überlegen. Die kurze Messzeit der DPOAE erlaubt ein sehr dichtes Scanning der Innenohrfunktion, die mit herkömmlichen subjektiven Methoden überhaupt nicht oder nur mit für den Patienten unzumutbar langen Untersuchungszeiten möglich ist. Mit der hier beschriebenen Vorrichtung lassen sich DPOAE mit extrem hoher Frequenzauflösung (bis zu 2 Hz) messen. Nach der Frequenz-Orts-Karte des Innenohres entspricht ein Frequenzschritt von 2 Hz in etwa dem Abstand einer einzelnen Hörsinneszelle. Mit einer solch hohen Frequenzauflösung lassen sich auch kleinste Veränderungen der Innenohrfunktion nachweisen. Die hier beschriebene Apparatur eignet sich daher zur Erfassung beginnender Sinneszellschädigungen, insbesondere zur Früherkennung von berufsbedingten und nicht berufsbedingten Hörschäden durch Lärm, zur Erkennung einer erhöhten Vulnerabilität des Innenohres sowie zur Bewertung der Erholung von Sinneszellen (therapiebegleitende Verlaufskontrolle) beispielsweise bei Schädigungen infolge Lärmrexposition, bei Hörsturz oder bei Verabreichung ototoxischer Medikamente, wie z.B. bei der Chemotherapie.

*5. Tinnitusdiagnostik*

Die Frage, ob Tinnitus ein peripheres oder zentrales Geschehen ist, ist immer noch unbeantwortet. Nach neuesten Ergebnissen zeichnen sich erstmalig Möglichkeiten einer objektiven Diagnostik des cochleären Tinnitus mit Hilfe der DPOAE ab (Janssen et al. in "Growth behavior of the 2f1-f2 distortion product otoacoustic emission in tinnitus", J. Acoust. Soc. Am. 103 (6), Seiten 3418 bis 3430, Juni 1998). Hierbei wurde festgestellt, dass bei einigen Tinnituspatienten erhöhte Emissionspegel und versteilertes Wachstum auftreten. Dies ist Zeichen einer erhöhten Verzerrung der mikromechanischen Strukturen des Innenohres und kann als Ursache des Tinnitus gedeutet werden. Durch Aufnahme der DPOAE-Wachstumsfunktionen ermöglicht die vorliegende Apparatur eine verbesserte topologische Diagnostik des Tinnitus durch Erkennung von spezifischen Haarzellschädigungen als mögliche Ursache des cochleären Tinnitus. Erhöhte Pegel und versteuertes Wachstum der DPOAE im Frequenzbereich des Tinnitus werden dabei als Hinweis auf eine cochleäre Genese des Tinnitus gewertet.

Da es sich bei den eingesetzten Methoden um objektive Verfahren handelt, ist die aktive Mitarbeit des Patienten nicht erforderlich. Der Patient soll vor der Messung über den Messablauf informiert und instruiert werden, wie er sich während der Messung verhalten soll. Die Instruktionen über die adäquate Verhaltensweise (ruhige Sitzposition einnehmen etc.) und den Messablauf (Dauer der Messung etc.) können vorzugsweise auf Compakt Disk (CD) oder ähnlichen Medien gespeichert und dem Patienten über die Ohrsonde zugespielt werden.

Durch eine automatische Artefakterkennung können bei kurzzeitiger Unruhe des Patienten die gestörten Signalabschnitte verworfen und die Aufnahme des jeweiligen Messdatums wiederholt werden. Bei zu langer Unruhe wird die Messung automatisch gestoppt und der Patient zur Ruhigstellung aufgefordert. Bei Patienten mit starken Atemgeräuschen (z.B. behinderte Nasenatmung bei Schnupfen) wird eine geeignete Artefakterkennung eingesetzt, die eine störungsfreie Messung in den Atempausen ermöglicht.

Zur Beschleunigung des Messablaufs kann eine an ein Abbruchkriterium (z.B. Signal-Störabstand) gebundene Mittelung der Daten erfolgen. Während der Messung sind die Reizparameter (Schallpegel der Primärtöne und des Gaußimpuls) und die Reiz- und Ableitbedingungen (Kalibrierung des Schalldrucks im Gehörgang, Elektrodenwiderstand) fortlaufend zu kontrollieren. Bei zu großen Abweichungen ist die Messung abzubrechen. Der Untersucher wird dann durch ein optisches oder akustisches Signal aufgefordert, die Störung (z.B. Herausfallen der Sonde, Lösen der Elektroden) zu beheben.

Der Signal-Störabstand beeinflusst die Stabilität des Emissionspegels. Die an einem Normkollektiv ermittelte Streubreite des Emissionspegels als Funktion des Signal-Störabstands soll als Grundlage dienen, eine statistische Aussage über die Güte der individuellen Messung zu treffen. Für eine erleichterte Befunderhebung werden die Messdaten in geeignete Kennlinienfelder eingetragen, um im pathologischen Fall das Ausmaß der Abweichung von der Norm zu kennzeichnen.

Neben den Methoden der konventionellen subjektiven Audiometrie kann mit der vorliegend beschriebenen Vorrichtung durch Applikation von transienten Schallreizen auch eine Registrierung der transitorisch evozierten otoakustischen Emissionen (TEOAE) erfolgen, wie sie beispielsweise in EP-B-0 015 258 beschrieben ist.

Durch rechnergestützte Datenanalyse und Qualitätssicherung der Messgrößen wird eine weitgehend automatisierte Diagnose-Stellung erreicht.

Die hier beschriebene Vorrichtung erlaubt eine allgemeine und eine im speziellen auf die Hörgeräteanpassung ausgerichtete Innenohrdiagnostik. Aus den Kenngrößen der gestörten Innenohrfunktion können Hörgeräteparameter abgeleitet werden, die zu einer verbesserten Anpassung von Hörgeräten führt. Als Folge der automatischen Messablaufsteuerung und automatischen Bereitstellung von Hörgeräteparametern ist auch eine automatisierte Anpassung von Hörgeräten möglich.

**Patentansprüche**

1. Verfahren zur Erfassung sowohl schwellennaher als auch überschwelliger Schallverarbeitung des Innenohres durch Messung von mindestens Distorsionsprodukten otoakustischer Emissionen DPOAE, wobei im Zuge des Verfahrens:

(a) eine Ohrsonde in den Gehörgang des zu untersuchenden Ohres eingeführt wird;
(b) ein erster Primärton mit einer Frequenz $f_i$ und einem Schallpegel $L_1$, sowie ein zweiter Primärton mit einer Frequenz $f_2$, die größer als die Frequenz $f_1$ des ersten Primärtones ist, und einem Schallpegel $L_2$ von der Ohrsonde im äußeren Gehörgang erzeugt werden, um DPOAE auszulösen, wobei der erste und der zweite Primärton mit einem Frequenzverhältnis $f_2/f_1$ mit Werten im Bereich von 1,05 bis 1,6 und Schallpegeln im äußeren Gehörgang des zu untersuchenden Ohres erzeugt werden, welche die Beziehung (I)

$$L_I = \sum_{n=0}^{N} a_n L_2{}^n \qquad\qquad \textbf{(I)}$$

erfüllen, wobei die Parameter so eingestellt werden, dass für einen gegebenen Schallpegel $L_2$ der Schallpegel $L_1$ so bestimmt wird, dass ein Emissionspegel $L_{DP}$ der gemessenen DPOAE maximal wird.
(c) Distorsionsprodukte otoakustischer Emissionen DPOAE im äußeren Gehörgang gemessen werden;
(d) die Frequenz $f_{DP}$ und der Schalldruck $p_{DP}$ bzw. der Emissionspegel $L_{DP}$ der gemessenen DPOAE ermittelt werden;

(e) der Schalldruck $p_{DP}$ bzw. der Emissionspegel $L_{DP}$ gegenüber dem Schallpegel $L_1$ bzw. $L_2$ von einem der Töne aufgetragen wird, um eine DPOAE-Wachstumsfunktion zu erhalten; und

(f) durch Extrapolation der DPOAE-Wachstumsfunktion in der Form $p_{DP}$ $(L_1, L_2)$ derjenige Schallpegel $L_s$ bestimmt wird, der zu einem Schwellenwert des Emissionsschalldruckes $p_{DP}$ z.B. von Null führt, um mit Hilfe dieses Schallpegels $L_s$ die Hörschwelle abzuschätzen.

2. Verfahren nach Anspruch 1, **dadurch gekennzeichnet, dass** der erste und der zweite Primärton mit Schallpegeln im äußeren Gehörgang des zu untersuchenden Ohres erzeugt werden, welche die Beziehung (I)

$$L_1 = \sum_{n=0}^{N} a_n L_2^{\,n} \qquad\qquad\qquad\qquad (I)$$

mit den Parametern $N = 1$, $25 \leq a_o \leq 50$ und $0{,}3 \leq a_1 \leq 0{,}6$ erfüllen.

3. Verfahren nach einem der vorhergehenden Ansprüche, **dadurch gekennzeichnet, dass** zur Extrapolation der DPOAE-Wachstumsfunktion eine vorzugsweise lineare Regressionsanalyse durchgeführt wird.

4. Verfahren nach einem der vorhergehenden Ansprüche, **dadurch gekennzeichnet, dass** das Kompressionsverhältnis k, bzw. die Steigung $s=1/k$, gemäß der nachstehenden Formel (III)

$$k = \frac{\Delta L_X}{\Delta L_{DP}} \qquad\qquad\qquad\qquad (III)$$

wobei x = 1 oder 2, vorzugsweise 2, ermittelt und vorzugsweise als Funktion $k(f)$ der Frequenz einer der Töne aufgetragen wird, um eine Messgröße für die quantitative Erfassung des Recruitments bereitzustellen.

5. Verfahren nach einem der vorhergehenden Ansprüche, **dadurch gekennzeichnet, dass** der Primärtonschwellenpegel $L_s$ als Hörschwelle und der Emissionspegel $L_{DP}$ als Iso-Pegelkurven im konventionellen Tonschwellenaudiogramm eingetragen werden, um eine gemeinsame Darstellung der schwellennahen Schallverarbeitungsmechanismen, Hörschwelle, als auch der überschwelligen Schallverarbeitungsmechanismen, Dynamikkompression, zu ermöglichen.

6. Verfahren nach einem der vorhergehenden Ansprüche, **dadurch gekennzeichnet, dass** mittels der Ohrsonde zusätzlich zu dem ersten und dem zweiten Ton ein dritter Ton, Suppressorton, mit einer Frequenz $f_{sup}$ und einem Schallpegel $L_{sup}$ im äußeren Gehörgang des zu untersuchenden Ohres erzeugt wird.

7. Verfahren nach Anspruch 6, **dadurch gekennzeichnet, dass** die Frequenz $f_{sup}$ des dritten Tones variiert wird und der Schallpegel $L_{sup}$, der zu einer Suppression des Emissionspegels $L_{DP}$ um einen bestimmten Wert führt, ermittelt wird, um eine DPOAE-Iso-Suppressionstuningkurve mit den Kenngrößen Steigung der hoch- und tieffrequenten Flanke $s_{tf}$ und $s_{hf}$, $Q_{10dB}$-Wert, charakteristische Frequenz $f_{CF}$ und Schwellenpegel $L_{DPCF}$ zu erhalten

8. Verfahren nach einem der vorhergehenden Ansprüche, **dadurch gekennzeichnet, dass** mittels der Ohrsonde FAEP auslösende Schallreize im äußeren Gehörgang erzeugt werden und frühe auditorisch evozierte Potentiale FAEP an der Kopfhaut der Person mit Elektroden abgegriffen werden.

9. Verfahren nach Anspruch 8 **dadurch gekennzeichnet, dass** die FAEP wahlweise simultan bei Auslösung der DPOAE mit dem ersten und dem zweiten Ton in der Einschaltphase oder nicht simultan mit transienten Schallreizen, Klick, Tonpip ausgelöst und gemessen werden.

10. Verfahren nach einem der vorhergehenden Ansprüche, **dadurch gekennzeichnet, dass** das Verhältnis der Frequenz $f_1$ des ersten Primärtones zu der Frequenz $f_2$ des zweiten Primärtones variiert wird und der Schalldruck $p_{DP}$ oder der Emissionspegel $L_{DP}$ als Funktion dieses Frequenzverhältnisses $f_2/f_1$ aufgetragen wird, um eine $f_2/f_1$-Tuningkurve zu erhalten, wobei $f_2/f_1$ zwischen 1,05 und 1,6 in Schritten im Bereich von 100 Hz bis 1 Hz variiert wird.

**11.** Verfahren nach einem der vorhergehenden Ansprüche, **dadurch gekennzeichnet, dass** die erfassten DPOAE-Signale, und bei Rückbeziehung auf einen der Ansprüche 8 bis 10 vorzugsweise auch die erfassten FAEP-Signale, mittels drahtloser Datenübertragung einer Auswerteanordnung zugeleitet werden.

**12.** Verfahren nach einem der vorhergehenden Ansprüche, **dadurch gekennzeichnet, dass** aus den ermittelten Messgrößen Kenngrößen abgeleitet und eine automatisierte Verlaufskontrolle bei Lärmexposition, Hörsturz, Verabreichung von Ototoxen, eine automatisierte Bestimmung von Hörschwelle, Trennschärfe und Lautheitsanstieg, Recruitment, und eine automatisierte Anpassung von Hörgeräten bewerkstelligt werden.

**13.** Verfahren nach einem der vorhergehenden Ansprüche, **dadurch gekennzeichnet, dass** mittels der Ohrsonde Signale im äußeren Gehörgang erzeugt werden, welche transitorisch evozierte otoakustische Emissionen TEOAE auslösen, und die TEOAE mit dem mindestens einen Mikrophon (20) registriert werden.

**14.** Vorrichtung zur Ausführung des Verfahrens nach einem der vorhergehenden Ansprüche, versehen mit:

einer tragbaren Einheit (10) mit:

mindestens einer Ohrsonde (12) mit
mindestens zwei Schallsendern (14, 16, 18) zur Applikation der die DPOAE auslösenden Schallreize gemäß Merkmal (b) von Anspruch 1; und
mindestens einem Mikrophon (20) zur Erfassung von Distorsionsprodukten otoakustischer Emissionen (DPOAE) gemäß Merkmal (c) von Anspruch 1; und
einer Stromversorgung (36);

einer stationären Einheit (22) umfassend:

einer Steueranordnung zur Steuerung des Messvorgangs; und
einer Auswerteanordnung (22, 32) zur Auswertung der von dem Mikrophon erfassten DPOAE gemäß den Merkmalen (d) bis (f) von Anspruch 1; sowie

einem Sende- und Empfangssystem (24, 26) zur drahtlosen Übertragung von Daten zwischen der tragbaren Einheit (10) und der stationären Einheit (22).

**15.** Vorrichtung nach Anspruch 14, **dadurch gekennzeichnet, dass** die Ohrsonde (12) eine Ohrolive (46) aufweist, die in den Gehörgang eines zu untersuchenden Ohrs derart einpassbar ist, dass es diesen dichtend abschließt.

**16.** Vorrichtung nach Anspruch 14 oder 15, **gekennzeichnet durch** mindestens eine Elektrodenanordnung (30) zur zusätzlichen Erfassung von frühen auditorisch evozierten Potentialen FAEP.

**17.** Vorrichtung nach einem der Ansprüche 14 bis 16, **dadurch gekennzeichnet, dass** die tragbare Einheit (10) eine Verstärkeranordnung (32) zur Verstärkung der von dem Mikrophon (20) erfassten DPOAE-Signale sowie gegebenenfalls der mittels der Elektrodenanordnung (30) erfassten FAEP-Signale umfasst.

**18.** Vorrichtung nach einem der Ansprüche 14 bis 17, **dadurch gekennzeichnet, dass** die tragbare Einheit (10) eine Digitalisierungseinrichtung (34) zur Digitalisierung der erfassten Signale aufweist.

**19.** Vorrichtung nach einem der Ansprüche 14 bis 18, **dadurch gekennzeichnet, dass** das Sende- und Empfangssystem (26) ausgelegt ist, Daten mittels Infrarot- oder Funkübertragung zu übertragen.

**20.** Vorrichtung nach einem der Ansprüche 14 bis 19, **dadurch gekennzeichnet, dass** die tragbare Einheit (10) als ein am Kopf eines Probanden zu tragender Halter, insbesondere als Kopf oder Kinnbügel, Headset, ausgelegt ist.

**21.** Vorrichtung nach einem der Ansprüche 14 bis 20, **gekennzeichnet durch** Mittel zum Anlegen von Signalen, die transitorisch evozierten otoakustische Emissionen TEOAE auslösen, an mindestens einen der Schallsender.

**22.** Vorrichtung nach einem der Ansprüche 14 bis 21, **dadurch gekennzeichnet, dass** die Ohrsonde (12) einen dritten Schallsender (18) zur Abgabe eines Suppressortones aufweist.

**23.** Vorrichtung nach Anspruch 22, **dadurch gekennzeichnet, dass** die Ohrsonde (12) mehrere parallelgeschaltete Mikrophone aufweist.

**24.** Vorrichtung nach einem der Ansprüche 14 bis 23, **dadurch gekennzeichnet, dass** die Stromversorgung (36) eine nachladbare Stromquelle aufweist.

**25.** Vorrichtung nach einem der Ansprüche 14 bis 24, **dadurch gekennzeichnet, dass** ein tragbarer Signalgeber (44) vorgesehen ist, der eine Eingabevorrichtung sowie ein Sendesystem zur drahtlosen Übertragung von mittels der Eingabevorrichtung erzeugten Signalen zu der stationären Einheit (22) aufweist.

**26.** Vorrichtung nach einem der Ansprüche 14 bis 25, **dadurch gekennzeichnet, dass** die stationäre Einheit (22) eine erste Speicher- und Wiedergabeanordnung zur Speicherung und Wiedergabe von während eines Messvorgangs ermittelten Daten aufweist.

**27.** Vorrichtung nach einem der Ansprüche 14 bis 26, **dadurch gekennzeichnet, dass** die stationäre Einheit (22) eine zweite Speicher- und Wiedergabeanordnung zur Speicherung und Wiedergabe eines Programms zur Steuerung eines Messvorgangs aufweist.

**28.** Vorrichtung nach einem der Ansprüche 14 bis 27, **dadurch gekennzeichnet, dass** zwei Ohrsonden (12) vorgesehen sind.

**Claims**

**1.** Method for evaluating sound processing both near and above the auditory threshold by measuring at least distortion products of otoacoustic emissions DPOAE, in which method:

(a) an ear probe is inserted into the auditory canal of the ear to be examined;
(b) a first primary tone having a frequency $f_1$ and a sound level $L_1$ and a second primary tone having a frequency $f_2$ that is larger than the frequency $f_1$ of the first primary tone and a sound level $L_2$ are generated by means of the ear probe within the outer auditory canal, so as to initiate DPOAE, wherein the first and second primary tones are generated within the outer auditory canal of the ear to be examined so as to have to frequency ratio $f_2/f_1$ with values in the range of from 1.05 to 1.6 and sound levels which fulfil relation (I)

$$L_1 = \sum_{n=0}^{N} a_n L_2{}^n \qquad\qquad (I)$$

wherein the parameters are adjusted such that for a given sound level $L_2$ the sound level $L_1$, is determined such that an emission level $L_{DP}$ of the measured DPOAE reaches a maximum;
(c) distortion products of otoacoustic emissions DPOAE are measured within the outer auditory canal;
(d) the frequency $f_{DP}$ and the sound level $p_{DP}$ or the emission level $L_{DP}$, respectively, of the measured DPOAE are determined;
(e) the sound pressure $p_{DP}$ or the emission level $L_{DP}$, respectively, are plotted over the sound level $L_1$, or $L_2$, respectively, of one of the tones so as to obtain a DPOAE growth function; and
(f) by extrapolating the DPOAE growth function in the form of $p_{DP}(L_1, L_2)$ a sound level $L_s$ is determined, which results in a threshold level of the emission sound pressure $p_{DP}$ of, for example, zero, so as to estimate the hearing threshold.

**2.** Method of claim 1, **characterized in that** the first and the second primary tone are generated within the outer auditory canal of the ear to be examined which fulfil the relation (I)

$$L_1 = \sum_{n=0}^{N} a_n L_2{}^n \qquad\qquad (I)$$

with the parameters $N = 1$, $25 \le a_0 \le 50$ and $0.3 \le a_1 \le 0.6$.

3. Method according to anyone of the preceding claims, **characterized in that** for extrapolation of the DPOAE growth function a preferably linear regression analysis is performed.

4. Method according to anyone of the preceding claims, **characterized in that** the compression ratio k or the gradient s = 1/$k$, respectively, is determined in accordance with below formula (III)

$$k = \frac{\Delta L_x}{\Delta L_{DP}} \qquad \text{(III)}$$

wherein x = 1 or 2, preferably 2, and preferably is plotted as function $k(f)$ of the frequency of one of the tones, so as to provide for a measured value for the quantitative evaluation of the recruitement.

5. Method according to anyone of the preceding claims, **characterized in that** the sound level of the primary tone $L_s$ is recorded as hearing level and the emission level $L_{DP}$ is recorded as isolevel curves in a conventional sound threshold audiogram, so as to enable simultaneous illustration of the sound processing mechanisms near the threshold, hearing threshold, and also above the threshold, dynamic compression.

6. Method according to anyone of the preceding claims, **characterized in that** by means of the ear probe, in addition to the first and the second tones, a third tone, suppressor tone, having a frequency of $f_{sup}$ and a sound level $L_{sup}$ is generated within the outer auditory canal of the ear to be examined.

7. Method according to claim 6, **characterized in that** the frequency $f_{sup}$ of the third tone is varied and the sound level $L_{sup}$ which results in a suppression of the emission level $L_{DP}$ by a certain value is determined, so as to obtain a DPOAE-iso-suppression tuning curve with the characteristic parameters gradient of the high-frequency and low frequency flank $s_{tf}$ and $_{shf}$, $Q_{10dB}$ value, characteristic frequency $f_{CF}$ and threshold level $L_{DPCF}$.

8. Method according to anyone of the preceding claims, **characterized in that** by means of the ear probe sound stimuli evoking FAEP are generated within the outer auditory canal and brainstem auditory evoked potentials FAEP are measured at the scalp of a person by means of electrodes.

9. Method according to claim 8, **characterized in that** the FAEP selectively are evoked and measured simultaneously with evoking the DPOAE with the first and the second tone during the start-up phase, or non-simultaneously with transient sound stimuli, click, tonpip.

10. Method according to anyone of the preceding claims, **characterized in that** the ratio of the frequency $f_1$ of the first primary tone and the frequency $f_2$ of the second primary tone is varied and the sound pressure $p_{DP}$ or the emission level $L_{DP}$ is plotted as a function of the frequency ratio $f_2/f_1$, so as to obtain a $f_2/f_1$ tuning curve, wherein $f_2/f_1$ is varied between 1.05 and 1.6 in steps in the region of from 100 Hz to 1 Hz.

11. Method according to anyone of the preceding claims, **characterized in that** the measured DPOAE signals, and when dependent on one of claims 8 to 10, preferably also the measured FAEP signals, are transmitted by means of wireless data transmission to an evaluation means.

12. Method according to anyone f the preceding claims, **characterized in that** parameters are derived from the determined measurement values and an automated progress evaluation of noise exposition, sudden deafness, administration of oto-toxic substances, an automated determination of the hearing threshold, selectivity and loudness increase, recruitment, and an automated adaptation of hearing aids is accomplished.

13. Method according to anyone of the preceding claims, **characterized in that** by means of the ear probe signals are generated within the outer auditory canal which evoke transiently evoked otoacoustic emissions TEOAE, and wherein the TEOAE are recorded with at least one microphone (20).

14. Apparatus for carrying-out the method according to anyone of the preceding claims, comprising:

a portable unit (10) comprising:

at least one ear probe (12) having:

at least two sound sources (14, 16, 18) for application of the sound stimuli evoking DPOAE in accordance with feature (b) of claim 1; and
at least one microphone (20) for registering the distortion products of otoacoustic emissions (DPOAE) in accordance with feature (c) of claim 1; and

a power source (36);

a stationary unit (22) comprising:

a control means for control of the measuring procedure; and
evaluation means (22, 32) for evaluation of the DPOAE registered by means of the microphone in accordance with features (d) to (f) of claim 1; and

a transmitter and receiver system (24, 26) for wireless transmission of data between the portable unit (10) and the stationary unit (22).

15. Apparatus according to claim 14, **characterized in that** the ear probe (12) comprises an ear tip (46), which can be fitted into the auditory canal of the ear to be examined in a manner so as to sealingly close the latter.

16. Apparatus according to claim 14 or 15, **characterized by** at least one electrode arrangement (30) for additional registration of brainstem auditory evoked potentials FAEP.

17. Apparatus according to anyone of claims 14 to 16, **characterized in that** the portable unit (10) comprises amplifier means (32) for amplification of the DPOAE signals registered by means of the microphone (20) and optionally of the FAEP-signals registered by means of the electrode array (30).

18. Apparatus according to anyone of claims 14 to 17, **characterized in that** the portable unit (10) comprises digitizing means (34) for digitizing the registered signals.

19. Apparatus according to anyone of claims 14 to 18, **characterized in that** the transmission and receiver system (26) is designed to transmit data by means of infrared or radio transmission.

20. Apparatus according to anyone of claims 14 to 19, **characterized in that** the portable unit (10) is designed as a fixture to be worn on the head of a test person, particularly as a head bar or chin bar, headset.

21. Apparatus according to anyone of claims 14 to 20, **characterized by** means for applying signals which initiate transiently evoked otoacoustic emissions TEOAE to at least one of the sound sources.

22. Apparatus according to anyone of claims 14 to 21, **characterized in that** the ear probe (12) comprises a third sound source (18) which delivers a suppressor tone.

23. Apparatus according to claim 22, **characterized in that** the ear probe (12) comprises a plurality of microphones which are connected in parallel.

24. Apparatus according to anyone of claims 14 to 23, **characterized in that** the power supply (36) comprises a rechargeable power source.

25. Apparatus according to anyone of claims 14 to 24, **characterized in that** there is provided a portable signal generator (44) comprising input means and a transmitter system for wireless transmission of signals generated by means of the input means to the stationary unit (22).

26. Apparatus according to anyone of claims 14 to 25, **characterized in that** the stationary unit (22) comprises a first storage and retrieval means for storage and retrieval of data which were determined during the measuring operation.

**27.** Apparatus according to anyone of claims 14 to 26, **characterized in that** the stationary unit (22) comprises a second storage and retrieval means for storage and retrieval of a program for control of the measuring operation.

**28.** Apparatus according to anyone of claims 14 to 27, **characterized in that** there are provided two ear probes (12).

**Revendications**

**1.** Procédé pour l'enregistrement du traitement sonore proche du seuil ou supérieur au seuil de l'oreille interne par la mesure d'au moins des produits de distorsion d'émissions oto-acoustiques DPOAE, moyennant quoi, dans le cadre du procédé :

(a) une sonde auriculaire est introduite dans le conduit auditif de l'oreille à examiner ;
(b) un premier son primaire avec une fréquence $f_1$ et un niveau sonore $L_1$ ainsi qu'un second son primaire avec une fréquence $f_2$, qui est supérieure à la fréquence $f_1$ du premier son primaire, et un niveau sonore $L_2$ sont générés par la sonde auriculaire dans le conduit auditif externe, afin de déclencher des DPOAE, le premier et le second son primaire étant générés avec un rapport de fréquence $f_2/f_1$ présentant des valeurs comprises entre 1,05 et 1,6 et des niveaux sonores dans le conduit auditif externe de l'oreille à examiner, qui satisfont à la relation (I) suivante

$$L_1 = \sum_{n=0}^{N} a_n L_2^n \qquad \text{(I)}$$

les paramètres étant réglés de telle sorte que, pour un niveau sonore $L_2$ donné, le niveau sonore $L_1$ est déterminé de telle sorte qu'un niveau d'émission $L_{DP}$ des DPOAE mesurés devient maximum ;
(c) des produits de distorsion d'émissions oto-acoustiques DPOAE sont mesurés dans le conduit auditif externe ;
(d) la fréquence $f_{DP}$ et la pression sonore $p_{DP}$, ou le niveau d'émission $L_{DP}$ des DPOAE mesurés sont déterminés ;
(e) la pression sonore $p_{DP}$ ou le niveau d'émission $L_{DP}$ est reporté par rapport au niveau sonore $L_1$ ou $L_2$ de l'un des sons, afin d'obtenir une fonction de croissance DPOAE ; et
(f) le niveau sonore $L_s$, qui aboutit à une valeur seuil de la pression sonore d'émission $p_{DP}$ par exemple de zéro, est déterminé par l'extrapolation de la fonction de croissance DPOAE sous la forme $p_{DP}$ $(L_1, L_2)$ afin d'évaluer le seuil auditif à l'aide de ce niveau sonore $L_S$.

**2.** Procédé selon la revendication 1, **caractérisé en ce que** le premier et le second son primaire sont générés avec des niveaux sonores dans le conduit auditif externe de l'oreille à examiner, qui satisfont à la relation (I) suivante

$$L_1 = \sum_{n=0}^{N} a_n L_2^n \qquad \text{(II)}$$

avec les paramètres $N = 1$, 25 $a_0 \leq 50$ et $0,3 \leq a_1 \leq 0,6$.

**3.** Procédé selon l'une quelconque des revendications précédentes, **caractérisé en ce qu'**une analyse de préférence par régression linéaire est effectuée pour l'extrapolation de la fonction de croissance DPOAE.

**4.** Procédé selon l'une quelconque des revendications précédentes, **caractérisé en ce que** le rapport de compression $k$, ou la pente s=1/$k$, est déterminé selon la formule (III) suivante

$$k = \frac{\Delta Lx}{\Delta L_{DP}} \qquad \text{(III)}$$

x étant égal à 1 ou 2, de préférence à 2, et étant appliqué de préférence comme fonction $k(f)$ de la fréquence de l'un des sons, afin de mettre à disposition une grandeur de mesure pour l'enregistrement quantitatif de l'égalisation de l'intensité du son.

**5.** Procédé selon l'une quelconque des revendications précédentes, **caractérisé en ce que** le niveau de seuil du son primaire $L_s$ comme seuil auditif et le niveau d'émission $L_{DP}$ sont reportés sous forme de courbes de niveau Iso dans le diagramme de seuil sonore classique afin de permettre une représentation conjointe des mécanismes de traitement du son proche du seuil, seuil auditif, ainsi que des mécanismes de traitement du son supérieure au seuil, compression de dynamique.

**6.** Procédé selon l'une quelconque des revendications précédentes, **caractérisé en ce qu'**un troisième son, son de suppression, avec une fréquence fsup et un niveau sonore Lsup est généré dans le conduit auditif externe de l'oreille à examiner au moyen de la sonde auriculaire en supplément du premier et du second son.

**7.** Procédé selon la revendication 6, **caractérisé en ce que** la fréquence fsup du troisième son est modifiée et le niveau sonore Lsup, qui aboutit à une suppression du niveau d'émission $L_{DP}$ d'une valeur définie, est déterminé afin d'obtenir une courbe de syntonisation de suppression Iso de DPOAE avec les paramètres pente du flanc à haute fréquence et du flanc à basse fréquence $S_{if}$ et $S_{hf}$, valeur $Q_{10dB}$, fréquence caractéristique $f_{CF}$ et niveau seuil $L_{DPCF}$.

**8.** Procédé selon l'une quelconque des revendications précédentes, **caractérisé en ce que** des stimulations acoustiques déclenchant des FAEP sont générées dans le conduit auditif externe au moyen de la sonde auriculaire et des potentiels précoces évoqués au niveau auditif FAEP sont prélevés sur le cuir chevelu de la personne avec des électrodes.

**9.** Procédé selon la revendication 8, **caractérisé en ce que** les FAEP sont déclenchés et mesurés au choix simultanément en cas de déclenchement des DPOAE avec le premier et le second son dans la phase d'enclenchement ou de façon non simultanée avec des stimulations sonores transitoires, clic, bip sonore.

**10.** Procédé selon l'une quelconque des revendications précédentes, **caractérisé en ce que** le rapport de la fréquence $f_1$ du premier son primaire à la fréquence $f_2$ du second son primaire est modifié et la pression sonore $p_{DP}$ ou le niveau d'émission $L_{DP}$ est reporté en fonction de ce rapport de fréquence $f_2/f_1$, afin d'obtenir une courbe de syntonisation $f_2/f_1$, $f_2/f_1$ étant modifié entre 1,05 et 1,6 par incréments dans la plage de 100 Hz à 1 Hz.

**11.** Procédé selon l'une quelconque des revendications précédentes, **caractérisé en ce que** les signaux DPOAE enregistrés et en cas de référence à l'une des revendications 8 à 10, de préférence également les signaux FAEP enregistrés sont acheminés à un dispositif d'analyse au moyen de transmission de données sans fil.

**12.** Procédé selon l'une quelconque des revendications précédentes, **caractérisé en ce que** des grandeurs caractéristiques sont déduites des grandeurs mesurées déterminées et qu'un contrôle de tracé automatisé en cas d'exposition au bruit, de perte brutale de l'audition, d'administration d'ototoxes, d'une détermination automatisée du seuil auditif, de la sélectivité et de l'augmentation du niveau sonore, de l'égalisation de l'intensité du son, et une adaptation automatisée des prothèses auditives sont réalisés.

**13.** Procédé selon l'une quelconque des revendications précédentes, **caractérisé en ce que** des signaux sont générés dans l'appareil auditif externe au moyen de la sonde auriculaire, lesquels déclenchent des émissions oto-acoustiques évoquées de façon transitoire TEOAE, et les TEOAE sont enregistrées avec le au moins un microphone (20).

**14.** Dispositif pour la mise en oeuvre du procédé selon l'une quelconque des revendications précédentes, équipé de :

    une unité (10) portative comportant :

        au moins une sonde auriculaire (12) avec
        au moins deux émetteurs sonores (14, 16, 18) pour l'application des stimulations sonores déclenchant les DPOAE selon la caractéristique (b) de la revendication 1 ; et
        au moins un microphone (20) pour l'enregistrement de produits de distorsion d'émissions oto-acoustiques (DPOAE) selon la caractéristique (c) de la revendication 1 ; et
        une alimentation électrique (36) ;

    une unité (22) stationnaire comportant :

        un dispositif de commande pour la commande de l'opération de mesure ; et

un dispositif d'analyse (22, 32) pour l'analyse des DPOAE enregistrés par le microphone selon les caractéristiques (d) à (f) de la revendication 1 ; et

un système d'émission et de réception (24, 26) pour la transmission sans fil de données entre l'unité (10) portative et l'unité (22) fixe.

15. Dispositif selon la revendication 14, **caractérisé en ce que** la sonde auriculaire (12) présente une olive auriculaire (46) qui peut être adaptée dans le conduit auditif d'une oreille à examiner de telle sorte qu'elle le ferme de façon étanche.

16. Dispositif selon la revendication 14 ou 15, **caractérisé par** au moins un dispositif d'électrodes (30) pour l'enregistrement supplémentaire de potentiels précoces évoqués de façon auditive FAEP.

17. Dispositif selon l'une quelconque des revendications 14 à 16, **caractérisé en ce que** l'unité (10) portable comporte un dispositif amplificateur (32) pour l'amplification des signaux DPOAE enregistrés par le microphone (20) et éventuellement des signaux FAEP enregistrés au moyen du dispositif d'électrodes (30).

18. Dispositif selon l'une quelconque des revendications 14 à 17, **caractérisé en ce que** l'unité (10) portable présente un dispositif de numérisation (34) pour la numérisation des signaux enregistrés.

19. Dispositif selon l'une quelconque des revendications 14 à 18, **caractérisé en ce que** le système d'émission et de réception (26) est conçu pour transmettre des données au moyen de transmission infrarouge ou transmission radio.

20. Dispositif selon l'une quelconque des revendications 14 à 19, **caractérisé en ce que** l'unité (10) portable est conçue comme un support à porter sur la tête d'un sujet, en particulier sous forme de serre-tête ou de mentonnière, casque audio.

21. Dispositif selon l'une quelconque des revendications 14 à 20, **caractérisé par** des moyens pour l'application de signaux, qui déclenchent des émissions oto-acoustiques évoquées de façon transitoire TEOAE sur au moins l'un des émetteurs de son.

22. Dispositif selon l'une quelconque des revendications 14 à 21, **caractérisé en ce que** la sonde auriculaire (12) présente un troisième émetteur sonore (18) pour l'émission d'un son de suppression.

23. Dispositif selon la revendication 22, **caractérisé en ce que** la sonde auriculaire (12) présente plusieurs microphones branchés en parallèle.

24. Dispositif selon l'une quelconque des revendications 14 à 23, **caractérisé en ce que** l'alimentation électrique (36) présente une source électrique rechargeable.

25. Dispositif selon l'une quelconque des revendications 14 à 24, **caractérisé en ce qu'**un générateur de signaux (44) portable est prévu, qui présente un dispositif d'entrée et un système d'émission pour la transmission sans fil de signaux générés au moyen du dispositif d'entrée à l'unité (22) fixe.

26. Dispositif selon l'une quelconque des revendications 14 à 25, **caractérisé en ce que** l'unité (22) fixe présente un premier dispositif de stockage et de restitution pour le stockage et la restitution de données déterminées pendant une opération de mesure.

27. Dispositif selon l'une quelconque des revendications 14 à 26, **caractérisé en ce que** l'unité (22) fixe présente un second dispositif de stockage et de restitution pour le stockage et la restitution d'un programme pour la commande d'une opération de mesure.

28. Dispositif selon l'une quelconque des revendications 14 à 27, **caractérisé en ce que** deux sondes auriculaires (12) sont connues.

**FIG. 1**

# FIG. 2

**DP-Gramme**

L1=65..67, L2=65..20 dB SPL

$L_{DP}$ [dB SPL]

20
10
0
-10
-20
-30

0,5    1    2    4    8

$f_2$ [kHz]

**Audiogramm**

Hörverlust [dB HL]

0
20
40
60
80
100
120

125    500 1k 2k 4k 8k

f [Hz]

**Steigungsprofil**

s [dB/dB]

1.5
1
0.5
0

0,5    1    2    4    8

$f_2$ [kHz]

**Wachstumsfunktion**

$L_{DP}$ [dB SPL]

20
10
0
-10
-20
-30

f2= 2002 Hz

s = y / x

y

x

20 30 40 50 60

$L_2$ [dB SPL]

DPOAE-Iso-Suppressionstuningkurve

$Q_{10\,dB} = f_{CF} / bb$

**FIG. 3**

DPOAE-Wachstumsfunktion

**FIG. 4**

## DPOAE-Wachstumsfunktionen

FIG. 5

**FIG. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0015258 B
- GB 2205403 A
- US 5664577 A, Lonsbury-Martin
- DE 19549165 A

**In der Beschreibung aufgeführte Nicht-Patentliteratur**

- **JANSSEN et al.** Growth behavior of the 2f1-f2 distortion product otoacoustic emission in tinnitus. *J. Acoust. Soc. Am.,* Juni 1998, vol. 103 (6), 3418-3430
- **KUMMER et al.** The level and growth behavior of the 2f1-f2 distortion product otoacoustic emission and its relationship to auditory sensitivity in normal hearing and cochlear hearing loss. *J. Acoust. Soc. Am.,* Juni 1998, vol. 103 (6), 3431-3444
- **KUMMER et al.** Suppression tuning characteristics of the 2f1-f2 distortion product otoacoustic emission in humans. *J. Acoust. Soc. Am.,* Juli 1995, vol. 98 (1), 197-210